# EUROPÄISCHE PATENTANMELDUNG

(11) **EP 1 092 416 A2**
(43) Veröffentlichungstag der Anmeldung: **18.04.2001**
(21) Anmeldenummer: 00122161.3
(22) Anmeldetag: 12.10.2000
(51) Int. Cl.: A61K 7/02, A61K 7/043

(54) **Verwendung feinteiliger, farbstoffhaltiger Polymerisate PF als farbgebender Bestandteil in kosmetischen Mitteln**

(30) Priorität: 13.10.1999 DE 19949382
(71) Anmelder: BASF AKTIENGESELLSCHAFT, 67056 Ludwigshafen (DE)
(72) Erfinder: Medelnick, Monika, 67065 Ludwigshafen (DE); Pfrommer, Ellen, 67454 Hassloch (DE); Clemens, Thorsten, 67126 Hochdorf-Assenheim (DE); Erk, Peter, 67227 Frankenthal (DE); Böhm, Arno, 68305 Mannheim (DE); Kielhorn-Bayer, Sabine, 67133 Maxdorf (DE); Witteler, Helmut, 67259 Beindersheim (DE); Dausch, Wilma M., 67117 Limburgerhof (DE); Westenfelder, Horst, 67435 Neustadt (DE); Wünsch, Thomas, 67346 Speyer (DE); Mathauer, Klemens, 67063 Ludwigshafen (DE); Habeck, Thorsten, 97149 Meckenheim (DE); Ikeda, Takahiro, Odawara 250-0874 (JP); Ichihara, Hideyuki, Odawara, Kanagawa 250-0874 (JP)
(74) Vertreter: Kinzebach, Werner, Dr.

(57) **Zusammenfassung**

Die vorliegende Erfindung beschreibt die Verwendung feinteiliger, farbstoffhaltiger Polymerisate PF in Form einer wässrigen Polymerdispersion oder eines daraus erhältlichen Polymerpulvers, dessen Polymermatrix wenigstens einen organischen Farbstoff F homogen verteilt enthält, als farbgebenden Bestandteil in kosmetischen Mitteln.

Die vorliegende Erfindung beschreibt auch kosmetische Mittel, die ein farbstoffhaltiges Polymerisat in einer Menge von 0,1 bis 50 Gew.-%, bezogen auf das Gesamtgewicht des kosmetischen Mittels, sowie für kosmetische Mittel übliche Zusätze enthalten.

## Beschreibung

Die vorliegende Erfindung betrifft die Verwendung feinteiliger, farbstoffhaltiger Polymerisate PF als farbgebenden Bestandteil in kosmetischen Mitteln.

Kosmetische Mittel werden in der Regel zum Schutz der Haut, insbesondere der Gesichtshaut, der Haare, der Finger- und der Fußnägel vor mechanischen Einwirkungen, vor Austrocknen und vor Infektionen, verwendet. Bei einer Reihe von kosmetischen Mitteln soll neben der pflegenden und schützenden Wirkung auch eine optische Wirkung erzielt werden. Zum Teil steht die optische Wirkung sogar im Vordergrund. Beispiele für kosmetische Mittel, mit denen eine optische Wirkung erzielt werden soll, sind Mittel zur Behandlung der Gesichtshaut, wie Kajal-Stifte, Eyeliner, Lidschatten, Make-up-Formulierungen, Tönungscremes, Theaterschminken, Haarbehandlungsmittel, wie Wet-Gels, Haargels mit Glimmerlook, Stylinggels, Haarspray, Haarmascara, ferner Nagellacke und Sonnenschutzformulierungen, wie Sun-Block-Cremes und Sun-Block-Stifte. Kosmetika, bei denen die optische Wirkung im Vordergrund steht, werden häufig auch als dekorative Kosmetika bezeichnet.

Dekorative Kosmetika enthalten in der Regel organische oder anorganische Pigmente als farbgebenden Bestandteil. Diese werden in der Regel in die Kosmetika bei deren Herstellung eingearbeitet. Aufgrund ihrer Unlöslichkeit verhalten sich die Pigmente gegenüber den übrigen Bestandteilen des kosmetischen Mittels im Unterschied zu löslichen Farbstoffen weitgehend inert. Zudem hat die Unlöslichkeit der Pigmente den Vorteil, dass eine bleibende Verfärbung der Körperstellen, die mit dem kosmetischen Mittel behandelt wurden, vermieden werden kann.

Als nachteilig bei der Verwendung von Pigmenten erweist sich ihre im Vergleich zu organischen Farbstoffen geringere Farbbrillanz. Herkömmliche Pigmente müssen zudem vor ihrer Verwendung im kosmetischen Mittel aufgeschlossen werden, um eine Feinverteilung des Pigments im kosmetischen Mittel zu gewährleisten. Eine Feinverteilung des Pigments ist erforderlich, um eine gleichmäßige Färbung des kosmetischen Mittels zu garantieren und eine ausreichende Farbtiefe auch bei geringeren Einsatzmengen an Pigment zu erreichen. Zum Aufschließen des Pigmentes wird das Pigment in der Regel in einem Teil der öligen Phase zu einer pigmenthaltigen Paste angerieben, die dann bis zum gewünschten Feinheitsgrad gewalzt oder vermahlen wird. Dabei ist es erforderlich, dass die Farbpaste nicht zu dünn ist, damit ein guter Mahleffekt erreicht wird. Sie darf aber auch nicht zu fest sein, da sonst die Farbpaste nicht klumpenfrei in das kosmetische Mittel eingearbeitet werden kann.

Der vorliegenden Erfindung liegt somit die Aufgabe zugrunde, einen für kosmetische Mittel geeigneten farbgebenden Bestandteil bereitzustellen, der sich leicht in die kosmetischen Mittel einarbeiten lässt und zugleich die Vorteile der Pigmente, nämlich ein chemisch inertes Verhalten gegenüber den übrigen Bestandteilen der kosmetischen Zusammensetzung und eine geringe Wasserlöslichkeit, aufweist. Zudem sollte der farbgebende Bestandteil eine im Vergleich zu anorganischen oder organischen Pigmenten erhöhte Farbbrillanz aufweisen.

Es wurde nun überraschenderweise gefunden, dass diese Aufgabe gelöst wird, durch feinteilige, farbstoffhaltige Polymerisate PF, deren polymere Matrix wenigstens einen organischen Farbstoff F homogen verteilt enthält. Die feinteiligen Polymerisate PF werden dabei in Form ihrer wässrigen Polymerdispersion oder in Form eines daraus hergestellten Polymerpulvers eingesetzt.

Demnach betrifft die vorliegende Erfindung die Verwendung feinteiliger, farbstoffhaltiger Polymerisate PF in Form einer wässrigen Polymerdispersion oder eines daraus erhältlichen Polymerpulvers, dessen Polymermatrix wenigstens einen organischen Farbstoff homogen verteilt enthält, als farbgebenden Bestandteil in kosmetischen Mitteln.

Unter homogener Verteilung des organischen Farbstoffs versteht man, dass der organische Farbstoff in der polymeren Matrix des farbstoffhaltigen Polymerisats molekulardispers verteilt, d. h. monomolekular gelöst oder in Form von bi- oder höhermolekularen Farbstoffaggregaten gelöst vorliegt.

Der Begriff Farbstoff umfasst hier und im Folgenden chemische Verbindungen oder Salze chemischer Verbindungen sowie Charge-Transfer-Komplexe von chemischen Verbindungen mit einem Chromophor, der ein Absorptionsmaximum im Wellenlängenbereich von 400 bis 850 nm aufweist und somit für das menschliche Auge einen Farbeindruck hervorruft (konventionelle Farbstoffe) und der gegebenenfalls auch selber Licht im sichtbaren Bereich emittiert (Fluoreszenzfarbstoffe). Farbstoffe im Sinne dieser Erfindung sind auch Verbindungen mit einem Absorptionsmaximum im Bereich von 250 bis 400 nm, die bei Bestrahlen mit UV-Licht eine Fluoreszensstrahlung im sichtbaren Bereich emittieren (optische Aufheller). Farbstoffe im Sinne dieser Erfindung sind weiterhin chemische Verbindungen, die Licht der Wellenlänge < 400 nm absorbieren und strahlungslos deaktivieren (UV-Stabilisatoren bzw. UV-Absorber).

Erfindungswesentlich ist, dass sich der Farbstoff in der polymeren Matrix des farbstoffhaltigen Polymerisats homogen verteilen lässt. Dies ist in der Regel dann gewährleistet, wenn der organische Farbstoff, gegebenenfalls in Form eines Salzes, in den niedermolekularen Bestandteilen, welche die Polymermatrix bilden (Monomere), eine zumindest begrenzte Löslichkeit aufweist. Vorzugsweise weist der organische Farbstoff eine Löslichkeit auf, die größer ist als die geplante Einsatzmenge im Polymeren. Geeignete Farbstoffe F weisen insbesondere eine Löslichkeit > 0,5 Gew.-%, insbesondere > 1 Gew.-% und ganz besonders bevorzugt > 5 Gew.-% in den Monomeren auf.

Je nach Farbintensität des Farbstoffs enthält das farbstoffhaltige Polymerisat PF in der Regel wenigstens 0,1 Gew.-%, bezogen auf das Gewicht der Polymermatrix, vorzugsweise 0,5 bis 50 Gew.-%, insbesondere 1 bis 30 Gew.-% und besonders bevorzugt 5 bis 30 Gew.-% wenigstens eines organischen Farbstoffs F.

In der Regel weisen die monomerlöslichen Farbstoffe F keine ionischen funktionellen Gruppen auf. Der Farbstoffchromophor wird einen oder mehrere Substituenten aufweisen, welche die Löslichkeit der Farbstoffe in den unpolaren oder wenig polaren Monomeren verbessern. Geeignete Substituenten sind beispielsweise C₁-C₁₀-Alkyl, das gegebenenfalls durch Heteroatome unterbrochen und/oder durch Hydroxy und/oder Halogen substituiert sein kann, C₁-C₁₀-Alkoxy, Amino, C₁-C₁₀-Alkylamino, Bis-C₁-C₁₀-Dialkylamino, C₁-C₁₀-Alkylaminocarbonyl, C₁-C₁₀-Alkyloxycarbonyl, C₁-C₁₀-Alkylcarbonylamino, C₁-C₁₀-Alkylcarbonyloxy, C₆-C₁₀-Aryl, C₆-C₁₀-Aryloxy, C₆-C₁₀-Arylcarbonyl, C₆-C₁₀-Arylcarbonyloxy, C₆-C₁₀-Arylamino, C₆-C₁₀-Arylcarbonylamino, C₆-C₁₀-Aryloxycarbonyl, C₆-C₁₀-Arylaminocarbonyl, C₆-C₁₀-Aryl-C₁-C₄-Alkyl, Heterocyclyl, Halogen und Nitro.

Unter C₁-C₁₀-Alkyl ist hier und im Folgenden sowohl lineares oder verzweigtes Alkyl mit 1 bis 10 Kohlenstoffatomen zu verstehen, das durch ein oder mehrere, nicht benachbarte Sauerstoffatome, Schwefelatome, Iminogruppen oder Alkyliminogruppen unterbrochen und/oder ein oder mehrfach durch Halogen oder Hydroxy substituiert sein kann. Beispiele für unsubstituierte Alkylgruppen umfassen Methyl, Ethyl, n-Propyl, i-Propyl, n-Butyl, 2-Butyl, Isobutyl, tert.-Butyl, n-Pentyl, Isopentyl, Neopentyl, tert.-Pentyl, Hexyl, 2-Methylpentyl, Heptyl, 1-Ethylpentyl, n-Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl, Eicosyl. Beispiele für durch Sauerstoff unterbrochene Alkylgruppen umfassen 2-Meth-oxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Isopropoxypropyl, 2- oder 3-Butoxypropyl, 2- oder 3-(2-Ethylhexyl-oxypropyl) etc. Halogensubstituierte Alkylgruppen umfassen insbesondere Perfluoralkylgruppen mit 1 bis 4 C-Atomen, wie Trifluormethyl. Beispiele für Hydroxy-substituierte Alkylgruppen sind Hydroxymethyl, 1- oder 2-Hydroxyethyl, 1-, 2- oder 3-Hydroxypropyl, 1,2-Bishydroxyethyl.

Unter C₆-C₁₀-Aryl versteht man Phenyl oder Naphthyl, das gegebenenfalls durch 1, 2 oder 3 Substituenten, ausgewählt unter C₁-C₄-Alkyl, C₁-C₄-Alkyloxy, Halogen, Hydroxy, das gegebenenfalls auch ethoxyliert sein kann, substituiert ist. C₆-C₁₀-Aryl-C₁-C₄-alkyl steht für C₆-C₁₀-Aryl, das durch eine C₁-C₄-Alkylengruppe gebunden ist. Beispiele für C₁-C₄-Alkylen umfassen Methylen, 1,1-Ethylen, 1,2-Ethylen, 1,1-Propylen, 1,2-Propylen, 2,2-Propylen, 1,3-Propylen und 1,4-Butylen. Beispiele für C₆-C₁₀-Aryl-C₁-C₄-alkyl umfassen insbesondere Benzyl und 2-Phenylethyl, die gegebenenfalls auch substituiert sind.

Beispiele für Cycloalkyl sind aliphatische Monocyclen wie Cyclopentyl, Cyclohexyl und Cycloheptyl sowie aliphatische Polycyclen wie Norbornyl, Adamantyl oder Decahydronaphthyl, die wie Aryl substituiert sein können. Unter Heterocycloalkyl versteht man aliphatische mono- und polycyclische Reste, die wenigstens ein Heteroatom, z. B. S, O und/oder N, im Ring aufweisen. Beispiele hierfür sind Pyrrolidinyl, Furanyl, Piperidinyl, Oxazolidinyl, Morpholinyl und Tetrahydropyranyl. Unter Heteroaryl versteht man Reste, die sich von gegebenenfalls substituierten oder benzanellierten Heteroaromaten wie Thiophen, Pyrrol, Pyrazol, Imidazol, Oxazol, Thiazol, Pyridin, Pyrimidin, Pyridazin, Triazin, Chinolin, Chinazolin u. ä. ableiten.

Beispiele für monomerlösliche, neutrale Farbstoffe F sind die gemäß Colour-Index als Disperse-Farbstoffe und die als Solvent-Farbstoffe bezeichneten Verbindungen, die auch als Dispersionsfarbstoffe bezeichnet werden. Eine Zusammenstellung geeigneter Dispersionsfarbstoffe findet sich beispielsweise in Ullmanns Enzyklopädie der technischen Chemie, 4. Auflage, Bd. 10, S. 155-165 (siehe auch Bd. 7, S. 585ff - Anthrachinonfarbstoffe; Bd. 8, S. 244ff - Azofarbstoffe; Bd. 9, S. 313ff - Chinophthalonfarbstoffe). Auf diese Literaturstelle und die darin genannten Verbindungen wird hiermit ausdrücklich Bezug genommen.

Erfindungsgemäß geeignete Dispersionsfarbstoffe und Solvent-Farbstoffe umfassen verschiedenste Farbstoffklassen mit unterschiedlichen Chromophoren, beispielsweise Anthrachinonfarbstoffe, Monoazo- und Disazofarbstoffe, Chinophthalone, Methin- und Azamethinfarbstoffe, Naphthalimidfarbstoffe, Naphthochinonfarbstoffe und Nitrofarbstoffe. Beispiele für erfindungsgemäß geeignete Dispersionsfarbstoffe sind die Dispersionsfarbstoffe der folgenden Colour-Index Liste:
C. I. Disperse Yellow 1, 2, 3, 4, 5, 6, 7, 8, 9, 10, 11, 11:1, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119. 120, 121, 179, 180, 181, 182, 183, 184, 184:1, 200, 201, 202, 203, 204, 205, 206, 207, 208, 209, 210, 211, 212, 213, 214, 215, 216, 217, 218, 219, 220, 221, 222, 223, 224, 225, 226, 227, 228.
C. I. Disperse Orange 1, 2, 3, 3:3, 4, 5, 6, 7, 8, 9, 10, 11, 12, 13, 14, 15, 16, 17, 18,19, 20, 21, 22, 23, 24, 25, 25:1, 26, 27, 28, 29, 30, 31, 32, 33, 34, 35, 36, 37, 38, 39, 40, 41, 41:1, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 126, 127, 128, 129, 130, 131, 136, 137, 138, 139, 140, 141, 142, 143, 145, 146, 147, 148.
C. I. Disperse Red 1, 2, 3, 4, 5, 5:1, 6, 7, 8, 9, 10, 11, 12, 13, 14, 15, 16, 17, 18, 19, 20, 21, 22, 23, 24, 25, 26, 27, 28, 29, 30, 30:1, 31, 32, 33, 34, 35, 36, 38, 39, 40, 41, 43, 43:1, 46, 48, 50, 51, 52, 53, 54, 55, 55:1, 56, 58, 59, 60, 61, 63, 65, 66, 69, 70, 72, 73, 74, 75, 76, 77, 79, 80, 81, 82, 84, 85, 86, 86: 1, 87, 88, 89, 90, 91, 92, 93, 94, 96, 97, 98, 100, 102, 103, 104, 105, 106, 107, 108, 109, 110, 111, 112, 113, 115, 116, 117, 118, 120, 121, 122, 123, 125, 126, 127, 128, 129, 130, 131, 132, 133, 134, 135, 136, 137, 138, 139, 140, 141, 142, 143, 144, 145, 146, 147, 148, 149, 150, 151, 151:1, 152, 153, 154, 155, 156, 157, 158, 159, 160, 161, 162, 163, 164, 165, 166, 167, 167:1, 168, 169, 170, 171, 172, 173, 174, 175, 176, 177, 178, 179, 180, 181, 182, 183, 184, 185, 186, 187, 188, 189, 190, 190:1, 191, 191:1, 192, 193, 194, 195, 211, 223, 273, 274, 275, 276, 277, 278, 279, 280, 281, 302:1, 305, 306, 307, 308, 309, 310, 311, 312, 313, 314, 315, 316, 317, 318, 319, 320, 321, 322, 323, 324, 325, 326, 327, 328 329, 330, 331, 332, 333, 334, 335, 336, 338, 339, 340, 341, 342, 343, 344, 346, 347, 348, 349.
C. I. Disperse Violet 1, 2, 3, 4, 4:1, 5, 6, 7, 8, 9, 10, 11, 12, 13, 14, 15, 16, 17, 18, 19, 20, 21, 22, 23, 24, 25, 26, 27, 28, 29, 31, 33, 34, 35, 36, 37, 38, 39, 40, 41, 42, 43, 44, 45, 46, 47, 48, 49, 50, 51, 52, 53, 54, 55, 56, 57, 58, 59, 60, 81, 86, 87, 88, 89, 91, 92, 93, 94, 95, 96, 97.
C. I. Disperse Blue 1, 1:1, 2, 3, 3:1, 4, 5, 6, 7, 7:1, 8, 9, 10, 11, 12, 13, 13:1, 14, 15, 16, 17, 18, 19, 20, 21, 22, 23, 23:1, 24, 25, 26, 27, 28, 29, 30, 31, 32, 33, 34, 35, 36, 38, 39, 40, 42, 43, 44, 45, 47, 48, 49, 51, 52, 53, 54, 55, 56, 58, 60, 60:1, 61, 62, 63, 64, 64:1, 65, 66, 68, 70, 72, 73, 75, 76, 77, 79, 80, 81, 81:1, 82, 83, 84, 85, 86, 87, 88, 89, 90, 91, 92, 93, 94, 95, 96, 97, 98, 99, 100, 101, 102, 103, 104, 105, 106, 107, 108, 109, 111, 112, 113, 114, 115, 116, 117, 118, 119, 121, 122, 123, 124, 125, 126, 127, 128, 130, 131, 132, 133, 134, 136, 137, 138, 139, 140, 141, 142, 143, 144, 145, 146, 147, 148, 149, 150, 151, 152, 153, 154, 155, 156, 158, 159, 160, 161, 162, 163, 164, 165, 165:2, 166, 167, 168, 169, 170, 171, 172, 173, 174, 175, 195, 281, 282, 283, 283:1, 284, 285, 286, 287, 288, 289, 290, 291, 292, 293, 294, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 349.
C. I. Disperse Green 1, 2, 5, 6, 9.
C. I. Disperse Brown 1, 2, 3, 4, 4:1, 5, 7, 8, 9, 10, 11, 18, 19, 20, 21.
C. I. Disperse Black 1, 3, 4, 5, 6, 7, 8, 9, 10, 11, 12, 13, 14, 15, 20, 22, 24, 25, 26, 27, 28, 29, 29:1, 30, 31, 32, 33, 34, 36.

Beispiele für erfindungsgemäß geeignete Solvent-Farbstoffe sind die Verbindungen der folgenden Colour-Index Liste:
C. I. Solvent Yellow 2, 3, 7, 12, 13, 14, 16, 18, 19, 21, 25, 25:1, 27, 28, 29, 30, 33, 34, 36, 42, 43, 44, 47, 56, 62, 72, 73, 77, 79, 81, 82, 83, 83:1, 88, 89, 90, 93, 94, 96, 98, 104, 107, 114, 116, 117, 124, 130, 131, 133, 135, 141, 143, 145, 146, 157, 160:1, 161, 162, 163, 167, 169, 172, 174, 175, 176, 179, 180, 181, 182, 183, 184, 185, 186, 187, 189, 190, 191.
C. I. Solvent Orange 1, 2, 3, 4, 5, 7, 11, 14, 20, 23, 25, 31A , 40:1, 41, 45, 54, 56, 58, 60, 62, 63, 70, 75, 77, 80, 81, 86, 99, 102, 103, 105, 106, 107, 108, 109, 110, 111, 112, 113.
C. I. Solvent Red 1, 2, 3, 4, 8, 16, 17, 18, 19, 23, 24, 25, 26, 27, 30, 33, 35, 41, 43, 45, 48, 49, 52, 68, 69, 72, 73, 83:1, 84:1, 89, 90, 90:1, 91, 92, 106, 109, 111, 118, 119, 122, 124, 125, 127, 130, 132, 135, 141, 143, 145, 146, 149, 150, 151, 155, 160, 161, 164, 164:1, 165, 166, 168, 169, 172, 175, 179, 180, 181, 182, 195, 196, 197, 198, 207, 208, 210, 212, 214, 215, 218, 222, 223, 225, 227, 229, 230, 233, 234, 235, 236, 238, 239, 240, 241, 242, 243, 244, 245, 247, 248.
C. I. Solvent Violet 2, 8, 9, 11, 13, 14, 21, 21:1, 26, 31, 36, 37, 38, 45, 46, 47, 48, 49, 50, 51, 55, 56, 57, 58, 59, 60, 61.
C. I. Solvent Blue 2, 3, 4, 5, 7, 18, 25, 26, 35, 36, 37, 38, 43, 44, 45, 48, 51, 58, 59, 59:1, 63, 64, 67, 68, 69, 70, 78, 79, 83, 94, 97, 98, 99, 100, 101, 102, 104, 105, 111, 112, 122, 124, 128, 129, 132, 136, 137, 138, 139, 143.
C. I. Solvent Green 1, 3, 4, 5, 7, 28, 29, 32, 33, 34, 35.
C. I. Solvent Brown 1, 3, 4, 5, 12, 20, 22, 28, 38, 41, 42, 43, 44, 52, 53, 59, 60, 61, 62, 63.
C. I. Solvent Black 3, 5, 5:2, 7, 13, 22, 22:1, 26, 27, 28, 29, 34, 35, 43, 45, 46, 48, 49, 50.

Erfindungsgemäß geeignet sind weiterhin monomerlösliche Derivate des Naphthalins, des Anthracens, des Perylens, des Terrylens, des Quarterrylens, sowie monomerlösliche Diketopyrrolopyrrolfarbstoffe, Perinonfarbstoffe, Cumarinfarbstoffe, Isoindolin- und Isoindolinonfarbstoffe, Porphyrinfarbstoffe, Phthalocyanin- und Naphthalocyaninfarbstoffe.

Geeignete monomerlösliche Cumarinfarbstoffe sind beispielsweise in der US-A 3 880 869 und der DE-A 44 24 817 beschrieben, auf die hiermit in vollem Umfang Bezug genommen wird.

Geeignete unpolare Perylenfarbstoffe sind beispielsweise solche, wie sie in der US-A 4 618 694, der DE-A 24 51 782, der US-A 379 934, der US-A 4 446 324, der EP-A 277 980, der EP-A 657 436 oder der WO 96/22332 beschrieben sind. Weitere geeignete unpolare Perylenfarbstoffe lassen sich beispielsweise der EP-A 73 007 entnehmen. Auf die genannten Druckschriften wird hiermit in vollem Umfang Bezug genommen. Beispiele für bevorzugte Perylenfarbstoffe sind die 6,12-Dicyanoperylen-1,7-dicarbonsäure-C₂-C₁₀-alkylester, die Bis-(N-C₁-C₁₀-alkyl)perylentetracarbonsäurediimide und die entsprechenden N-(Alkylphenyl)-Verbindungen, die unter den Lumogen®F-Marken im Handel erhältlich sind (BASF Aktiengesellschaft, Deutschland), z. B. Lumogen®F Rot 300, Lumogen®F Gelb 083 und Lumogen®F Orange 240.

Geeignete Naphthalinfarbstoffe umfassen unter anderem Naphthalin-1,8-dicarbonsäureimide, die am Imid-Stickstoff mit unsubstituiertem, linearem oder verzweigtem C₁-C₂₀-Alkyl oder Aryl substituiert sind, und die in der 4- und/oder der 5-Position des Naphthalinrings C₁-C₆-Alkoxy-Substituenten aufweisen können.

Geeignete Anthracenfarbstoffe umfassen unter anderem 9,10-Diphenylanthracen, 9,10-Bisphenylethinylanthracen, 1,8-Dichloro-9,10-bisphenylethinylanthracen. Beispiele für geeignete Anthracenfarbstoffe lassen sich beispielsweise Ullmann's Encyclopedia of Industrial Chemistry, 5th Edition, Vol. A2, S. 402 f. entnehmen.

Geeignete Porphyrinfarbstoffe umfassen beispielsweise Tetraphenylporphyrin und Octaethylporphyrin sowie deren Zink- oder Nickelkomplexe.

Beispiele für geeignete Phthalocyaninfarbstoffe sind Metallophthalocyanine, insbesondere Kupferphthalocyanine, die an den Phenyleneinheiten des Chromophors löslichkeitsvermittelnde Alkylgruppen mit vorzugsweise 4 bis 20 C-Atomen aufweisen, wobei die Alkylreste direkt oder über eine funktionelle Gruppe, beispielsweise über eine Sulfonamidgruppe an den Chromophor gebunden sein können. Handelsüblich sind beispielsweise Tetra-C₄-C₁₀-alkylphthalocyanin-Komplexe, wie Tetra-tert-butylkupferphthalocyanin oder Tetra-n-octylkupferphthalocyanin, sowie Sulfonamide von ein oder mehrfach sulfonierten Metallophthalocyaninen mit C₁₀-C₂₀-Alkylaminen, z. B. das Tetrasulfonamid des vierfach sulfonierten Kupferphthalocyanins mit Stearylamin.

Erfindungsgemäß zählen zu den Farbstoffen F auch optische Aufheller, die in der Polymermatrix oder in den die Polymermatrix-konstituierenden Monomeren löslich, d. h. öllöslich, sind. Geeignete optische Aufheller sind beispielsweise Verbindungen der Klassen der Bisstyrylbenzole, der Stilbene, der Benzoxazole, der Cumarine, der Pyrene und der Naphthaline. Es ist möglich, die oben genannten Aufheller alleine oder auch als Mischungen untereinander anzuwenden.

Bei den oben genannten optischen Aufhellern handelt es sich in der Regel um an sich bekannte und handelsübliche Produkte. Sie sind beispielsweise in Ullmann's Encyclopedia of Industrial Chemistry, 5. Auflage, Band A18, S. 156-161, beschrieben oder können nach den dort genannten Methoden erhalten werden.

Zu den organischen Farbstoffen zählen definitionsgemäß auch UV-strahlenabsorbierende Verbindungen (UV-Stabilisatoren bzw. Absorber), die die absorbierte Strahlung strahlungslos deaktivieren. Derartige Verbindungen werden häufig als UV-Absorber in Sonnenschutzmitteln eingesetzt.

Zu den UV-Absorbern zählen substituierte Zimtsäureester (Cinnamate) wie Octyl-p-methoxycinnamat, Isopentyl-4-methoxycinnamat, Benzophenone, wie 4-Methoxy-2-hydroxybenzophenon-Sulfonsäure-Natriumsalz, Salicylate wie 4-Isopropylbenzylsalicylat, 4-Aminobenzoesäure und ihre Derivate wie ethoxilierter 4-Aminobenzoesäureethylester, 2-Ethylhexyl-4,4-dimethylaminobenzoesäure, Ester der 4,4-Diphenylbutadien-1,1-dicarbonsäure, z. B. deren Bis(2-ethylhexyl)ester, 2-Phenylbenzimidazol-4-sulfonsäure und deren Salze, Urocainsäure, deren Salz und deren Ester, Benzoxazole, Benzotriazole wie 2-(2H-Benzotriazol-2-yl)-4-methyl-6-(2-methyl-3-((1,1,3,3-tetramethyl-1-(trimethylsilyloxy)disiloxanyl)propyl)phenol, Benzylidencampher und seine Derivate, wie sie z. B. in der DE-A 3 836 630 genannt sind, z.B. 3-Benzylidencampher, 3(4'-Methylbenzyliden)d-1-campher, weiterhin α-(2-Oxoborn-3-yliden)toluol-4-sulfonsäure und ihre Salze, N,N,N-Trimethyl-4-(2-oxoborn-3-ylidenmethyl)anilinium-methosulfat, Dibenzoylmethane wie 4-tert.-Butyl-4'-methoxydibenzoylmethan, 2,4,6-Triaryltriazin-Verbindungen wie 2,4,6-Tris-{N-[4-(2-ethylhex-1-yl)oxycarbonylphenyl)amino}-1,3,5-triazin, 4,4'-((6-(((tert.-Butyl)aminocarbonyl)phenylamino)-1,3,5-triazin-2,4-diyl)imino)bis(benzoesäure-2'-ethylhexylester), 2-Cyano-3,3-diphenylacrylsäureethylester und -2'-ethylhexylester, sowie 2-Phenylbenzimidazol-5-sulfonsäure und ihre Salze. Weitere erfindungsgemäß geeignete UV-Absorber findet man in Cosmetic legislation, Vol.1, Cosmetic Products, European Commission 1999, S. 64-66, auf die hiermit Bezug genommen wird.

Weiterhin können die farbstoffhaltigen Polymerisate PF auch ionische Farbstoffe F als farbgebenden Bestandteil enthalten. Diese Farbstoffe sind als solche in der Regel nicht in der Polymermatrix löslich, können jedoch durch Derivatisierung nach bekanntem Verfahren in eine öllösliche, d. h. eine in der Polymermatrix lösliche Form (= Farbstoff F) überführt werden. Bei üblichen kationischen Farbstoffen können beispielsweise die Anionen gegen solche Anionen, die langkettige Alkylreste aufweisen, ausgetauscht werden. Zu den Anionen mit langkettigen Alkylresten zählen beispielsweise die Anionen langkettiger Carbonsäuren mit 8 bis 22 C-Atomen, Mono- und Dialkylphosphate mit 4 bis 22 C-Atomen je Alkylrest, Alkylsulfonate mit 8 bis 22 C-Atomen, z. B. Dodecylsulfonat. Entsprechend können Farbstoffe mit basischen Gruppen, die in der wässrigen Phase üblicherweise protoniert vorliegen, mit den Säuren der vorgenannten Anionen umgesetzt werden, wobei öllösliche Salze der Farbstoffe erhalten werden. Analog können Farbstoffe mit sauren funktionellen Gruppen bzw. mit anionischen Gruppen, z. B. Sulfat- oder Carboxylat-Gruppen, mit langkettigen Aminen oder Ammoniumsalzen, die wenigstens einen langkettigen organischen Rest aufweisen, in eine monomerlösliche Form überführt werden. Geeignete langkettige Carbonsäuren, bzw. deren Salze leiten sich von Fettsäuren, wie Caprinsäure, Palmitinsäure, Stearinsäure, Ölsäure, Linolsäure und Linolensäure ab. Geeignete Amine sind beispielsweise primäre, linear oder verzweigt tätige Alkylamine mit 8 bis 22 C-Atomen im Alkylrest.

Zur Überführung der wasserlöslichen, ionischen Farbstoffe in eine monomerlösliche Form reicht es in der Regel aus, den Farbstoff mit der langkettigen Säure, bzw. dem langkettigen Amin oder dem jeweiligen Salz der Säure bzw. des Amins zusammenzugeben. Gegebenenfalls wird man die Komponenten in einem Lösungsmittel oder den Monomeren suspendieren und bis zur Lösung der Farbstoffe erwärmen, wobei man üblicherweise rührt und/oder in Inertgasatmosphäre arbeitet. Geeignete wasserlösliche anionische Farbstoffe sind bekannt und im Handel erhältlich. Bei diesen Farbstoffen handelt es sich in der Regel um Farbstoffe der vorgenannten Farbstoffklassen, beispielsweise um Mono- oder Disazofarbstoffe, die jeweils mindestens eine Sulfonsäuregruppe aufweisen, um Sulfonsäuregruppen tragende Triarylmethanfarbstoffe, um Kupferphthalocyaninsulfonsäure, um sulfonsäuregruppenhaltige Chinolinfarbstoffe oder Stilbenfarbstoffe. Beispielhaft genannt seien die folgenden Nummern des Colour-Index:
Direct Yellow 4, 5, 11, 50, 127, 137, 147, 153;
Acid Orange 7, 8;
Direct Orange 15, 34, 102;
Direct Red 81, 239, 252-255;
Direct Violet 9, 51;
Acid Blue 9, 86;
Direct Blue 199, 218, 267, 273, 279, 281;
Acid Black 194, 208, 210, 221;
Direct Black 19, 161, 170 und 171.

Beispiele für kationische bzw. basische Farbstoffe umfassen beispielsweise Azo- und Disazofarbstoffe mit Aminogruppen oder Ammoniumgruppen, Triarylmethanfarbstoffe, oder Aminfarbstoffe, Methin- und Azamethinfarbstoffe, beispielsweise Basic Red 1, Basic Red 14, Basic Blue 7, Basic Blue 11, Basic Blue 26, Basic Violet 1, Basic Violet 4, Basic Violet 10 etc.

Weiterhin zählen zu den monomerlöslichen Farbstoffen F auch Komplexe aus basischen und sauren Farbstoffen bzw. Komplexe aus anionischen und kationischen Farbstoffen, beispielsweise der Komplex aus Chrysoidinbase und Metanilgelbsäure.

Die Art der Polymermatrix, die das farbstoffhaltige Polymerisat bildet, ist für die erfindungsgemäße Verwendung von untergeordneter Bedeutung. Die polymere Matrix kann sowohl von Polyestern als auch von Polyamiden, Polyurethanen oder Polymerisaten ethylenisch ungesättigter Monomere gebildet werden. Wesentlich ist, dass der Farbstoff in der polymeren Matrix homogen verteilt werden kann, und dass das farbstoffhaltige Polymerisat zur Bildung einer feinteiligen, wässrigen Dispersion geeignet ist.

Erfindungsgemäß geeignete, wässrige Dispersionen farbstoffhaltiger Polymerisate PF sowie die daraus hergestellten Polymerpulver sind aus dem Stand der Technik bekannt, beispielsweise aus der EP-A 691 390, der DE-A 4 436 892, der DE-A 19 521 500, der EP-A 566 448, der US 4,680,332, der EP-A 808 855, der WO 99/40123 sowie der älteren Anmeldung PCT/EP 99/07229. Auf die Offenbarung dieser Schriften zu wässrigen Dispersionen farbstoffhaltiger Polymerisate und den daraus hergestellten Polymerisatpulvern wird hiermit in vollem Umfang Bezug genommen.

Bei den farbstoffhaltigen Polymerisatdispersionen der EP-A 691 390 und der DE-A 4 436 892 handelt es sich um wässrige Polymerdispersionen, deren polymere Matrix aus ethylenisch ungesättigten Monomeren aufgebaut ist. Sie werden in der Regel hergestellt, indem man zunächst in einem organischen Lösungsmittel aus den ethylenisch ungesättigten Monomeren ein Polymer herstellt, in der Lösung des Polymeren einen organischen Farbstoff löst und die so erhaltene farbstoffhaltige Lösung in eine wässrige Dispersion überführt (sogenannte Sekundärdispersion). Die farbstoffhaltigen Polymerdispersionen der DE-A 19 521 500 werden in vergleichbarer Weise hergestellt, wobei anstelle des aus ethylenisch ungesättigten Monomeren aufgebauten Polymers ein Polyurethan eingesetzt wird.

Bei den farbstoffhaltigen, wässrigen Polymerisatdispersionen der EP-A 566 448 und der US 4,680,332 handelt es sich um Emulsionspolymerisate, die mit einer Lösung des organischen Farbstoffs in einem organischen Lösungsmittel imprägniert wurden.

Unter den aus dem Stand der Technik bekannten wässrigen Dispersionen farbstoffhaltiger Polymerisate PF sind solche bevorzugt, deren polymere Matrix aus ethylenisch ungesättigten Monomeren aufgebaut ist. Diese Polymerisate sind in der Regel ohne größeren Aufwand herstellbar und erlauben eine einfache Anpassung des Polymers an die Anforderungen des Farbstoffs, insbesondere im Hinblick auf dessen Löslichkeit in der Polymermatrix, sowie an die Anforderungen der kosmetischen Mittel. Bei den erfindungsgemäß zur Anwendung kommenden, wässrigen Dispersionen des farbstoffhaltigen Polymerisats hat es sich als günstig erwiesen, wenn die Polymerteilchen der Dispersion einen mittleren Teilchendurchmesser d_{z} im Bereich von 10 bis 1000 nm, vorzugsweise im Bereich von 50 bis 500 nm und insbesondere im Bereich von 50 bis 400 nm aufweisen. Bei dem mittleren Teilchendurchmesser d_{z} handelt es sich um den z-mittleren Teilchendurchmesser, bestimmt durch quasielastische, dynamische Lichtstreuung (berechnet durch unimodale Analyse der Autokorrelationsfunktion). Üblicherweise verwendet man zur Bestimmung des z-mittleren Teilchendurchmessers einen Coulter N4 Plus Particle Analyzer der Fa. Coulter Scientific Instruments. Die Messungen werden in der Regel an verdünnten, wässrigen Polymerdispersionen (0,01 gew.-%ig) bei Normalbedingungen (1 bar, 25 °C) vorgenommen. Ganz besonders bevorzugt liegt der Teilchendurchmesser im Bereich von 100 bis 300 nm. Ferner hat es sich als vorteilhaft erwiesen, wenn die Verteilung der Teilchengrößen der Polymerisatteilchen eng ist, d. h. das Verhältnis der Halbwertsbreite der durch die dynamische Lichtstreuung ermittelten Verteilungskurve der Polymerteilchengrößen zu der mittleren Teilchengröße klein ist. Das Verhältnis von verteilungsbreite zu mittlerer Teilchengröße d_{z} weist vorzugsweise Werte ≤ 1, insbesondere ≤ 0,75 und besonders bevorzugt ≤ 0,5 auf.

Weiterhin hat es sich als vorteilhaft erwiesen, wenn die Glasübergangstemperatur der polymeren Matrix T_{g} wenigstens 40 °C, vorzugsweise wenigstens 50 °C und insbesondere wenigstens 60 °C beträgt. Als Glasübergangstemperatur gilt hier und im Folgenden die nach ASTM D 3418-82 mittels Differenzialthermoanalyse bestimmte "Mid Point-Temperature". Eine Abschätzung der Glasübergangstemperatur für aus monoethylenisch ungesättigten Monomeren aufgebaute Polymere ist anhand der jeweiligen Monomerzusammensetzung mittels der Fox-Gleichung möglich (siehe Ullmann's Encyclopedia of Industrial Chemistry 5 ed, Vol. A21, S. 169). Eine hohe Glasübergangstemperatur ist insbesondere bei kosmetischen Mitteln vorteilhaft, deren Bestandteile bei der Herstellung aufgeschmolzen werden.

Unter den farbstoffhaltigen Polymerisaten PF, deren polymere Matrix aus monoethylenisch ungesättigten Monomeren M aufgebaut ist, sind insbesondere die aus der WO 99/40123 bekannten wässrigen, farbstoffhaltigen Polymerisatdispersionen sowie die daraus erhältlichen Polymerpulver besonders bevorzugt.

Derartige Polymerisate PF enthalten in Form ihrer wässrigen Dispersionen farbstoffhaltige Polymerisatteilchen mit einer mittleren Teilchengröße d₅₀ ≤ 1000 nm. Die Polymerteilchen sind aus ethylenisch ungesättigten Monomeren M aufgebaut und enthalten in ihrer polymeren Matrix wenigstens einen öllöslichen, d. h. in den Monomeren M zumindest begrenzt löslichen Farbstoff in homogener, d. h. molekulardisperser Verteilung. Zudem enthält die Polymermatrix derartiger Polymere häufig wenigstens ein vernetzendes Monomer einpolymerisiert.

Die in der WO 99/40123 beschriebenen wässrigen Dispersionen farbstoffhaltiger Polymerisate erfüllen häufig auch die zuvor angegebenen bevorzugten Anforderungen an die Teilchengröße und die Anforderungen an die Einheitlichkeit der Polymerisatteilchen. Zudem sind die organischen Farbstoffe in der Polymermatrix derartiger Polymerisatteilchen besonders gleichmäßig verteilt und auch besonders gut in der Polymermatrix gebunden. Wegen weiterer Details zu den farbstoffhaltigen Polymerisaten PF, den daraus hergestellten Polymerpulvern sowie zur Herstellung der farbstoffhaltigen, wässrigen Polymerisatdispersionen der Polymerisate PF und der Herstellung der Polymerpulver der farbstoffhaltigen Polymerisate PF sei auf die WO 99/40123 verwiesen, auf deren Offenbarung hiermit in vollem Umfang Bezug genommen wird.

Die polymere Matrix der aus der WO 99/40123 bekannten farbstoffhaltigen Polymerisate ist in der Regel aus ethylenisch ungesättigten Monomeren M aufgebaut, die wenigstens ein hydrophobes Monomer A mit einer Wasserlöslichkeit im Bereich von 0,01 bis 60 g/l, insbesondere 0,1 bis 50 g/l (bei 25 °C und 1 bar) umfassen. Die Monomere A machen in der Regel wenigstens 50 Gew.-%, vorzugsweise wenigstens 70 Gew.-% und insbesondere wenigstens 80 Gew.-%, bezogen auf das Gesamtgewicht der Monomere M aus.

Die Monomere A sind vorzugsweise ausgewählt unter
i) Estern von α,β-ethylenisch ungesättigten C₃-C₈-Monocarbonsäuren und C₄-C₈-Dicarbonsäuren mit C₁-C₈-Alkanolen oder C₅-C₈-Cycloalkanolen. Beispiele für diese Mono- und Dicarbonsäuren sind Acrylsäure, Methacrylsäure, Crotonsäure, Maleinsäure, Fumarsäure, Itaconsäure und Citraconsäure, wobei Acrylsäure und Methacrylsäure bevorzugt sind. Beispiele für C₁-C₈-Alkanole sind Methanol, Ethanol, n-Propanol, i-Propanol, n-Butanol, i-Butanol, sec-Butanol, t-Butanol, 2-Ethylhexanol und n-Octanol. Beispiele für C₅-C₈-Cycloalkanole sind Cyclopentanol und Cyclohexanol. Bevorzugte Ester sind Methylmethacrylat, n-Butylmethacrylat, Methylacrylat, Ethylacrylat, n-Butylacrylat, Cyclohexylacrylat und 2-Ethylhexylacrylat;
ii) Vinylestern von C₁-C₈-Monocarbonsäuren. Beispiele für Vinylester sind Vinylacetat, Vinylpropionat, Vinylbutyrat und Vinylhexanoat;
iii) vinylaromatischen Verbindungen, wie Styrol und α-Methylstyrol;
iv) C₂-C₆-Olefinen, wie Ethylen, Propen, 1-Buten, 2-Buten und Isobuten.

Es hat sich als vorteilhaft erwiesen, wenn die Monomere M neben den Monomeren A auch vernetzende bzw. vernetzend wirkende Monomere B umfassen. Durch die Monomere B wird eine bessere Einbindung des Farbstoffs in den farbstoffhaltigen Polymerisaten PF erreicht. Die Monomere B machen in der Regel 0,1 bis 30 Gew.-%, vorzugsweise 0,5 bis 20 Gew.-%, insbesondere 1 bis 10 Gew.-% der Monomere M aus.

Bei den Monomeren B handelt es sich insbesondere um Monomere B1, die wenigstens 2 nichtkonjugierte Doppelbindungen aufweisen. Derartige Monomere B1 werden, sofern erwünscht, in einer Menge von 0,1 bis 30 Gew.-%, bezogen auf die Gesamtmenge der zu polymerisierenden Monomere, insbesondere 0,5 bis 20 Gew.-% und ganz besonders bevorzugt 1 bis 10 Gew.-%, eingesetzt.

Geeignete Monomere B1 umfassen z. B. die Vinyl-, Allyl- und Methallylester der oben genannten ethylenisch ungesättigten Carbonsäuren ebenso wie die Ester dieser Säuren mit Tricyclodecenylalkohol, insbesondere die Ester der Methacrylsäure und der Acrylsäure, die Ester der oben genannten ethylenisch ungesättigten Carbonsäuren mit mehrwertigen Alkoholen, wie Ethylenglykoldiacrylat, Ethylenglykoldimethacrylat, Butandioldiacrylat, Butandioldimethacrylat, Hexandioldiacrylat, Hexandioldimethacrylat, Triethylenglykoldiacrylat, Trieethylenglykoltrimethacrylat, Tris(hydroxymethyl)ethantriacrylat und -trimethacrylat, Pentaerythrittriacrylat und -trimethacrylat, ferner die Allyl- und Methallylester von polyfunktionellen Carbonsäuren, wie Diallylmaleat, Diallylfumarat, Diallylphthalat. Typische Monomere B1 sind auch Verbindungen, wie Divinylbenzol, Divinylharnstoff, Diallylharnstoff, Triallylcyanurat, N,N'-Divinyl und N,N'-Diallylimidazolidin-2-on, sowie Methylenbisacrylamid und Methylenbismethacrylamid.

Zu den Monomeren B zählen auch monoethylenisch ungesättigte Verbindungen B2, die anstelle der wenigstens einen weiteren ethylenisch ungesättigten Bindung eine funktionelle Gruppe aufweisen, die zur nachträglichen Vernetzung des Polymerisats oder zu einer Reaktion mit einer funktionellen Gruppe des Farbstoffs, die nicht zum Chromophor des Farbstoffs gehört, in der Lage ist. Üblicherweise sind derartige funktionelle Gruppen ausgewählt unter Epoxy-, Hydroxy-, N-Methylol- oder Carbonylgruppen. Beispiele für Monomere B sind N-Alkylolamide der oben genannten ethylenisch ungesättigten Carbonsäuren, z. B. N-Methylol(meth)acrylamid, die Hydroxyalkylester der oben genannten ethylenisch ungesättigten Carbonsäuren, insbesondere Hydroxyethyl(meth)acrylat, die Bisacetonylamide der oben genannten ethylenisch ungesättigten Carbonsäuren, insbesondere N,N-Bisacetonyl(meth)acrylamid, ferner die Vinyl-, Allyl- und Methallylglycidylether, Glycidylester der oben genannten ethylenisch ungesättigten Carbonsäuren, wie Glycidyl(meth)acrylat, und weiterhin die Ester von Acetylessigsäure mit den Hydroxyalkylestern der oben genannten ethylenisch ungesättigten Carbonsäuren, z. B. Acetylacetoxyethyl(meth)acrylat.

Die genannten Monomere B2 können, sofern gewünscht, in Mengen von 0,1 bis 30 Gew.-%, vorzugsweise 0,5 bis 20 Gew.-%, insbesondere 1 bis 10 Gew.-%, bezogen auf die Gesamtmenge der zu polymerisierenden Monomere, mitpolymerisiert werden. In der Regel wird die Gesamtmenge an Monomeren B1 und B2 30 Gew.-%, vorzugsweise 20 Gew.-% und insbesondere 10 Gew.-%, bezogen auf die Gesamtmenge der Monomere M, nicht überschreiten.

Im Hinblick auf die Herstellbarkeit der erfindungsgemäßen farbstoffhaltigen wässrigen Polymerdispersionen der Polymerisate PF hat es sich außerdem als vorteilhaft erwiesen, wenn bei der Polymerisation der Monomere M besonders hydrophobe Monomere mit einer Wasserlöslichkeit < 0,01 g/l (bei 25 °C und 1 bar) zugegen sind (Monomere C). Monomere C werden, sofern erwünscht, in einer Menge von 0,1 bis 20 Gew.-%, insbesondere in einer Menge von 1 bis 10 Gew.-%, bezogen auf die Monomere M verwendet.

Beispiele für Monomere C, die eine wie vorstehend geforderte geringe wasserlöslichkeit aufweisen, sind 2- und 4-n-Butylstyrol, p-tert.-Butylstyrol, Ester aus 3 bis 6 C-Atome aufweisenden α,β-monoethylenisch ungesättigten Carbonsäuren und ≥ 12 C-Atome (in der Regel bis zu 30 C-Atome) aufweisenden Alkanolen wie z. B. Laurylacrylat und Stearylacrylat. Beispiele für Monomere C sind auch die Ester aus Vinylalkohol oder Allylalkohol und ≥ 9 C-Atome (in der Regel bis zu 30 C-Atome) aufweisenden Alkancarbonsäuren, wie z. B. Vinylnonanoat, Vinyldecanoat, Vinyllaurat und Vinylstearat, sowie im Handel befindliche Monomere VEOVA® 9-11 (VEOVA X ist ein Handelsname der Firma Shell und steht für Vinylester von Carbonsäuren, die auch als Versatic® X-Säuren bezeichnet werden). Zu den Monomeren C zählen auch Makromonomere wie Oligopropenacrylat (ganz allgemein sind Makromonomere polymere oder oligomere Verbindungen, die wenigstens eine, meist endständige, ethylenisch ungesättigte Doppelbindung aufweisen; ihr relatives zahlenmittleres Molekulargewicht sollte für eine Verwendbarkeit als geringst wasserlösliches Monomeres C vorzugsweise nicht mehr als 100000 betragen; in der Regel wird dieses relative zahlenmittlere Molekulargewicht 1000 bis 50000 bzw. 2000 bis 50000 betragen; Makromonomere sind dem Fachmann bekannt; ihre Herstellung ist beispielsweise in Makromol. Chem. 223 (1994) S. 29 bis 46 beschrieben). Ganz allgemein kommen als geringst wasserlösliche Monomere C alle diejenigen in Betracht, deren molare Löslichkeit bei 25 °C und 1 atm in Wasser gleich oder geringer als die entsprechende Löslichkeit von Laurylacrylat ist. Solche Monomeren C sind z. B. auch das Methacryloyl-Polybutylacrylat AB-6 und das Methacryloyl-Polystyrol A5-6 der Fa. Toa Gosei Kagaku KK (JP), die beide ein zahlenmittleres relatives Molekulargewicht von 6000 aufweisen. Aber auch Polyol 130 und Polyol 110 der Hüls AG (stereospezifisches, niedrigviskoses Polybutadien (75 % 1,4-cis, 24 % 1,4-trans, 1 % vinyl), dessen dynamische Viskosität bei 20 °C 3000 mPas beträgt) bilden als Makromonomere mit geringer Wasserlöslichkeit einsetzbare Monomere C. Anstelle der Polymeren C kann man auch nichtpolymerisierbare Verbindungen mit einer Wasserlöslichkeit < 0,01 g/l bei der Herstellung von Polymerisaten PF einsetzen.

Die Monomere M können weiterhin auch solche Monomere D umfassen, deren Homopolymerisate eine erhöhte Wasserlöslichkeit (d. h. > 60 g/l bei 25 °C) aufweisen. Derartige Monomere D dienen als modifizierende Monomere und werden - sofern erwünscht - in der Regel in Mengen ≤ 30 Gew.-%, bezogen auf die Gesamtmenge der zu polymerisierenden Monomere, vorzugsweise ≤ 20 und insbesondere ≤ 10 Gew.-%, beispielsweise in Mengen von 0,1 bis 20 Gew.-%, speziell 0,5 bis 10 Gew.-%, bezogen auf die zu polymerisierenden Monomere, verwendet. Zu den Monomeren D zählen sowohl monoethylenisch ungesättigte Monomere mit wenigstens einer Säuregruppe, z. B. einer COOH, SO₃H oder einer PO₃H₂-Gruppe, die auch in Salzform vorliegen kann (im Folgenden als anionische Monomere bezeichnet); monoethylenisch ungesättigte, kationische Monomere, insbesondere solche mit einer quartären Ammoniumgruppe (d. h. einer R₃N^{⊕}- Gruppe, worin R für Wasserstoff oder Alkyl, insbesondere Methyl oder Ethyl steht) oder einer Immoniumgruppe (d. h. einer =N^{⊕}(R)- Gruppe, worin R für Wasserstoff oder Alkyl, insbesondere Methyl oder Ethyl steht); und monoethylenisch ungesättigte, neutrale Monomere.

Beispiele für monoethylenisch ungesättigte anionische Monomere D (Monomere DA) sind 3 bis 6 C-Atome aufweisende, monoethylenisch ungesättigte Mono- und Dicarbonsäuren, z. B. Acrylsäure, Methacrylsäure, Maleinsäure, Itaconsäure, Acrylamidoglykolsäure, Methacrylamidoglykolsäure, Acryloyloxyglykolsäure, Methacryloyloxyglykolsäure, monoethylenisch ungesättigte Sulfonsäuren und Phosphonsäuren, z. B. Vinylsulfonsäure, Allylsulfonsäure, Methallylsulfonsäure, Styrolsulfonsäure, Vinylnaphthalinsulfonsäure und (Meth)acrylamido-2-methylpropansulfonsäure, weiterhin Vinylphosphonsäure, Allylphosphonsäure, Methallylphosphonsäure, Styrolphosphonsäure, und (Meth)acrylamido-2-methylpropanphosphonsäure, sowie deren wasserlösliche Salze, z. B. deren Alkalimetallsalze oder deren Ammoniumsalze, insbesondere deren Natriumsalze. Beispiele für neutrale Monomere D (Monomere DN) sind insbesondere die Amide monoethylenisch ungesättigter Mono- und Dicarbonsäuren, wie Acrylamid, Methacrylamid und Maleinimid, weiterhin N-Vinyllactame mit 3 bis 8 C-Atomen, wie N-Vinylpyrrolidon und N-Vinylcaprolactam sowie Acrylnitril. Geeignete kationische Monomere D (Monomere DK) sind insbesondere die Quaternisierungsprodukte und Protonierungsprodukte von monoethylenisch ungesättigten Aminen, beispielsweise die Quaternisierungsprodukte von Dialkylaminoalkylestern monoethylenisch ungesättigter Carbonsäuren, z. B. die Quaternisierungsprodukte von Dimethylaminoethylacrylat oder -methacrylat, und von Diethylaminoethylacrylat oder -methacrylat sowie die Quaternisierungsprodukte von 1-Vinylimidazolen wie 1-Vinylimidazol und 1-Vinyl-2-methylimidazol mit C₂-C₁₀-Oxiranen, C₁-C₁₀-Alkylhalogeniden oder C₁-C₁₀-Dialkylsulfaten, beispielsweise mit Methylhalogenid, Ethylhalogenid, Methylsulfat oder Ethylsulfat. Derartige Monomere sind beispielsweise aus der EP-A 246 580 und der US 4,859,756 bekannt. Die Quaternisierungsprodukte von 1-Vinylimidazolen werden im Folgenden auch als Vinylimidazolium-Salze, die Quaternisierungsprodukte von Aminoalkylacrylaten bzw. -methacrylaten als (Meth)acryloyloxyalkylammonium-Salze bezeichnet. Zu den Monomeren DN zählen auch Acrylnitril und Methacrylnitril, deren Einsatz häufig zu einer besseren Löslichkeit des Farbstoffs in den Monomeren M und damit zu einer besseren Verteilung des Farbstoffs in der polymeren Matrix führen. Acrylnitril und Methacrylnitril werden, sofern erwünscht, häufig in Mengen bis zu 50 Gew.-%, insbesondere in Mengen von 0,5 bis 30 Gew.-%, bezogen auf das Gesamtgewicht der Monomere M, bei der Herstellung der farbstoffhaltigen Polymerisate PF eingesetzt.

Unter den vorgenannten Polymerisaten werden für die erfindungsgemäße Verwendung insbesondere solche farbstoffhaltigen Polymerisate PF bevorzugt, worin 50 Gew.-% der Monomere A unter solchen Monomeren ausgewählt sind, deren Homopolymerisate eine Glasübergangstemperatur > 40 °C aufweisen (Monomere A1) und weniger als 50 Gew.-%, insbesondere weniger als 30 Gew.-% und besonders bevorzugt weniger als 10 Gew.-% solcher Monomere A, deren Homopolymerisate eine Glasübergangstemperatur < 40 °C aufweisen. Beispiele für besonders bevorzugte Monomere A1 sind Methylacrylat, Methylmethacrylat, Ethylmethacrylat, tert.-Butylacrylat, Vinylacetat, Styrol, Vinyltoluol und Methacrylnitril, wobei die vorgenannten Monomere A bis zu einer Menge von 50 Gew.-%, bezogen auf die Gesamtmonomermenge, durch Acrylnitril ersetzt sein können.

In einer ganz besonders bevorzugten Ausführungsform der erfindungsgemäßen, farbstoffhaltigen Polymerisate ist die polymere Matrix aufgebaut aus:
- 80 bis 99 Gew.-% Monomeren A, insbesondere Monomeren A1, besonders bevorzugt aus Methylacrylat, Styrol, Methylmethacrylat oder deren Mischungen, wobei bis zu 50 Gew.-% der Monomere A1 durch Acrylnitril ersetzt sein können,
- 1 bis 20 Gew.-%, insbesondere 2 bis 10 Gew.-%, Monomeren B, z. B. Divinylbenzol oder 1,4-Butandioldiacrylat,
- 0 bis 20 Gew.-%, z. B. 1 bis 20 Gew.-%, Monomeren C, z. B. Laurylacrylat oder Stearylacrylat, und
- 0 bis 20 Gew.-%, z. B. 1 bis 20 Gew.-%, Monomeren D, z. B. Acrylsäure, Methacrylsäure, Acrylamid, Methacrylamid, Acrylamido-2-methylpropansulfonat-Natriumsalz.

Die Herstellung der aus der WO 99/40123 bekannten, wässrigen Dispersionen farbstoffhaltiger Polymerisate PF erfolgt in der Regel durch radikalische wässrige Emulsionspolymerisation einer Öl-in-Wasser-Emulsion der Monomere M, worin die Monomertröpfchen (= Emulsionströpfchen) den Farbstoff in gelöster Form enthalten. Bevorzugt weisen die Tröpfchen einen mittleren Teilchendurchmesser d_{z} von ≤ 500 nm und insbesondere ≤ 400 nm auf. In der Regel wird d_{z} wenigstens 40 nm und vorzugsweise wenigstens 100 nm betragen. Die Tröpfchengröße der Öl-in-Wasser-Emulsion der Monomere wird ähnlich wie die Teilchengröße der Polymerisatteilchen des Polymerisats PF durch quasielastische, dynamische Lichtstreuung ermittelt. Vorzugsweise weisen die Tröpfchen in der farbstoffhaltigen Monomeremulsion eine weitgehend einheitliche Größe auf, d. h. der Quotient (d₉₀-d₁₀)/d₅₀ hat einen Wert ≤ 1, vorzugsweise ≤ 0,5, insbesondere ≤ 0,25. Hierin steht dₙ für den Teilchendurchmesser, den n Gew.-% der Emulsionströpfchen unterschreiten.

Zur Herstellung der farbstoffhaltigen Monomeremulsionen, worin die Emulsionströpfchen den Farbstoff gelöst oder molekulardispers verteilt enthalten, löst man zunächst den Farbstoff in den zu polymerisierenden Monomeren M. Die so erhaltene Farbstofflösung wird dann nach üblichen Methoden, beispielsweise durch Einrühren oder Dispergieren in einer wässrigen Lösung einer oberflächenaktiven Substanz, in eine Öl-in-Wasser-Emulsion überführt. Die so erhaltenen wässrigen Emulsionen weisen in der Regel mittlere Tröpfchengrößen d_{z} oberhalb von 1000 nm auf. Es hat sich bewährt, diese sogenannte "Makroemulsionen" in Monomeremulsionen mit Tröpfchengrößen ≤ 500 nm zu überführen. Die Polymerisation von Monomeremulsionen mit Tröpfchengrößen ≤ 500 nm führt zu besonders hochwertigen farbstoffhaltigen Polymerisaten PF. Derartige Monomeremulsionen werden auch als "Mini-Emulsionen" bezeichnet (vgl. P.L. Tang, E.D. Sudol, C.A. Silebi und M.S. El-Aasser in Journal of Applied Polymer Science, Vol. 43, S. 1059 - 1066 [1991]). Zu diesem Zweck werden vor der Polymerisation zu Mini-Emulsionen die konventionellen Monomeremulsionen vorzugsweise homogenisiert.

Die Homogenisierung erfolgt bevorzugt durch Anwendung von Ultraschall (z. B. Branson Sonifier II 450). Für die Homogenisierung mittels Ultraschall sind beispielsweise die in der GB 22 50 930 A und der US 5,108,654 beschriebenen Vorrichtungen geeignet. Die Anwendung von Ultraschall hat sich zur Herstellung der farbstoffhaltigen Mini-Emulsionen besonders bewährt und führt in der Regel zu besonders hochwertigen farbstoffhaltigen Polymerisaten PF. Demnach sind erfindungsgemäß solche Zubereitungen bevorzugt, worin das farbstoffhaltige Polymerisat PF durch Polymerisation einer Mini-Emulsion erhältlich ist, wobei die Mini-Emulsion erhältlich ist durch:
i) Lösen des Farbstoffs F in den Monomeren M1,
ii) Erzeugen einer konventionellen, farbstoffhaltigen Emulsion durch Emulgieren der Monomere in Gegenwart wenigstens einer oberflächenaktiven Verbindung, und
iii) Homogenisieren der konventionellen Emulsion mittels Ultraschall zu einer farbstoffhaltigen Mini-Emulsion.

Wegen Details zur Herstellung der Mini-Emulsionen wird an dieser Stelle auf die WO 99/40123 verwiesen, auf deren Offenbarung in vollem Umfang Bezug genommen wird.

Als oberflächenaktive Substanzen zur Herstellung der Emulsionen kommen grundsätzlich alle für die Emulsionspolymerisation bekannten, oberflächenaktiven Substanzen in Betracht, wobei neutrale, anionische und kationische Emulgatoren sowie neutrale, anionische oder kationische Schutzkolloide bevorzugt sind.

Die Art der oberflächenaktiven Substanz richtet sich wesentlich nach den Erfordernissen der kosmetischen Anwendung und ist im Hinblick auf die Herstellung von den farbstoffhaltigen Polymerisaten eher unkritisch.

Beispiele für Emulgatoren und Schutzkolloide, die sich sowohl für kosmetische Anwendungen als auch für die Herstellung der Polymerisate PF eignen, sind im Folgenden angegeben:

Anionische Emulgatoren sind beispielsweise Alkali- und Ammoniumsalze von Alkylsulfaten (Alkylrest: C₈ bis C₂₀), von Schwefelsäurehalbestern ethoxylierter Alkanole (EO-Grad: 4 bis 30, Alkylrest: C₁₀ bis C₂₀), von Schwefelsäurehalbestern ethoxylierter Alkylphenole (Alkylrest: C₄ bis C₉; EO-Grad: 3 bis 50), von sulfonierten Fettsäuren, von Di-C₄-C₂₀-alkylestern der Sulfobernsteinsäure und ähnliche Verbindungen.

Beispiele für geeignete neutrale Emulgatoren sind ethoxylierte Mono-, Di- und Tri-Alkylphenole (EO-Grad: 3 bis 50, Alkylrest: C₄ bis C₉), ethoxylierte Fettalkohole (EO-Grad: 3 bis 50, Alkylrest: C₈ bis C₃₆), ethoxylierte Oxoalkohole (EO-Grad: 3 bis 50, Alkylrest: C₈ bis C₃₆), ethoxylierte Glyceride von Fettsäuren (EO-Grad: 3 bis 50, Fettsäurerest).

Beispiele für kationische Emulgatoren sind Alkylammoniumhalogeniden mit wenigstens einer langkettigen Alkylgruppe, z. B. Dodecyltrimethylammoniumchlorid, -bromid, Cetyltrimethylammoniumchlorid oder -bromid, Dimethyl-C₁₂/C₁₄-alkylbenzylammoniumchlorid oder -bromid.

Weitere geeignete Emulgatoren finden sich in Houben-Weyl, Methoden der organischen Chemie, Band XIV/1, Makromolekulare Stoffe, Georg-Thieme Verlag, Stuttgart, 1961, S. 192-208, sowie in Ullmann's Encyclopedia of Industrial Chemistry, 5th ed., VCH Weinheim 1987, Vol. 9a, S. 313-318.

Schutzkolloide sind wasserlösliche, organische Polymere. Diese vermögen die Oberflächenspannung von Wasser kaum zu verringern und weisen im Unterschied zu Emulgatoren in der Regel oberhalb von 1000, vorzugsweise oberhalb 2000 liegende relative Molekulargewichte auf.

Beispiele für kationische Schutzkolloide sind die Homo- und Copolymere monoethylenisch ungesättigter kationischer Monomere beispielsweise Homo- und Copolymere von quaternisiertem N-Vinylimidazolen oder von quaternisierten Dialkylaminoalkylacrylaten und -methacrylaten mit beispielsweise N-Vinylpyrrolidon und/oder N-Vinylcaprolactam. Weiterhin zählen hierzu kationisch modifizierte Stärken.

Neutrale Schutzkolloide sind beispielsweise Polyvinylalkohole, teilverseifte Homo- und Copolymere des Vinylacetats, Ethylenoxid-Propylenoxid-Blockcopolymere, modifizierte Stärken, Cellulosederivate, Polyvinylpyrrolidon sowie Copolymere des Vinylpyrrolidons mit neutralen Monomeren z. B. mit Vinylcaprolactam, Vinylacetat, Acrylamid, Methacrylamid, Methylacrylat, Ethylacrylat, n-Butylacrylat oder mit Methylmethacrylat.

Beispiele für anionische Schutzkolloide sind Homo- und Copolymere monoethylenisch ungesättigter Carbonsäuren und deren Salze, insbesondere deren Natrium- und Ammoniumsalze, z. B. Homo- und Copolymere der Acrylsäure, der Methacrylsäure oder der Maleinsäure, gegebenenfalls mit neutralen Comonomeren, die z. B. ausgewählt sind unter den vorgenannten Monomeren A und den Monomeren D2, z. B. unter Styrol, Ester der (Meth)-Acrylsäure, Vinylacetat, Acrylamid, Methacrylamid, Acrylnitril und Hydroxyalkyl(meth)acrylaten. Anionische Schutzkolloide sind auch anionisch modifizierte Stärken und Stärkeabbauprodukte.

Die vorgenannten oberflächenaktiven Substanzen verbleiben naturgemäß nach der Herstellung in den wässrigen Dispersionen des farbstoffhaltigen Polymerisats PF und bestimmen dessen anwendungstechnischen Eigenschaften.

Die Herstellung dieser besonders bevorzugten wässrigen Polymerdispersionen der farbstoffhaltigen Polymerisate PF erfolgt dann durch Umsetzung der farbstoffhaltigen Emulsionen, vorzugsweise der oben beschriebenen Mini-Emulsionen mit einem radikalischen Polymerisationsinitiator. Dabei kann man so vorgehen, dass man die farbstoffhaltige Emulsion, vorzugsweise in Form einer Mini-Emulsion im Reaktor vorlegt und hierzu den Polymerisationsinitiator unter Polymerisationsbedingungen in einer Portion oder in mehreren Portionen oder kontinuierlich nach Maßgabe seines Verbrauchs zugibt. Auch kann man zunächst einen Teil oder die Gesamtmenge des Polymerisationsinitiators zu der Menge Emulsion geben und anschließend auf Polymerisationstemperatur erwärmen.

Es ist auch möglich, einen Teil oder die Gesamtmenge der wässrigen, farbstoffhaltigen Monomeremulsion, vorzugsweise in Form einer Mini-Emulsion, dem Polymerisationsgefäß unter Polymerisationsbedingungen nach Maßgabe des Fortschreitens der Umsetzung zuzugeben. Vorzugsweise führt man dabei den Polymerisationsinitiator zumindest teilweise parallel der Zugabe der Monomeremulsion dem Polymerisationsgefäß zu.

Als radikalische Polymerisationsinitiatoren kommen prinzipiell alle diejenigen in Betracht, die in der Lage sind, eine radikalische Polymerisation auszulösen. Es handelt sich hierbei sowohl um Peroxide, Hydroperoxide als auch um Azoverbindungen. Die radikalischen Polymerisationsinitiatoren können sowohl wasserlöslich sein als auch öllöslich, d. h. in den Monomeren löslich, sein.

Beispiele für wasserlösliche Initiatoren sind Peroxodischwefelsäure und ihre Ammonium- und Alkalimetallsalze, Wasserstoffperoxid oder niedermolekulare Hydroperoxide, wie tert.-Butylhydroperoxid, oder salzartige Azoverbindungen, z. B. 2,2'-Azobis-2-amidinopropan-dihydrohalogenid.

Beispiele für öllösliche Polymerisationsinitiatoren sind C₄-C₁₂-Peroxocarbonsäuren sowie ihre Ester, z. B. Peroctoate und Perbenzoate, wie tert.-Butylperoctoat und tert.-Butylperbenzoat sowie Diacylperoxide, wie Dibenzoylperoxid.

Die vorgenannten, wasserlöslichen peroxidischen Polymerisationsinitiatoren können auch mit einem Reduktionsmittel und gegebenenfalls mit einer im wässrigen Medium löslichen Metallverbindung kombiniert werden (sogenannten Redox-Initiatorsysteme). Diese sind dem Fachmann hinreichend bekannt. Wegen weiterer Details wird auf die WO 99/40123 verwiesen.

Die Menge an eingesetztem Initiator liegt in der Regel im Bereich von 0,1 bis 10 Gew.-%, vorzugsweise 0,2 bis 8 Gew.-% und häufig im Bereich von 0,3 bis 5 Gew.-%.

Bei der Herstellung der besonders bevorzugten, wässrigen Dispersionen der farbstoffhaltigen Polymerisate PF richten sich die Polymerisationstemperaturen in ersten Linie nach dem jeweils eingesetzten Redox-Initiatorsystem in bekannter Weise. Üblicherweise liegen die Polymerisationstemperaturen im Bereich von 0 bis 95 °C, vorzugsweise im Bereich von 30 bis 90 °C. Bei Anwendung von erhöhtem Druck kann die Polymerisationstemperatur auch bis zu 120 °C betragen. Üblicherweise wird bei Normaldruck (1 Atmosphären) polymerisiert.

Erfindungsgemäß kann das feinteilige, farbstoffhaltige Polymerisat anstelle in Form einer wässrigen Polymerisatdispersion auch in Form eines aus diesen wässrigen Polymerisatdispersionen erhältlichen Polymerpulvers eingesetzt werden. Verfahren zur Herstellung von Polymerpulvern aus wässrigen Polymerisatdispersionen sind dem Fachmann hinreichend bekannt. Die Herstellung erfolgt in der Regel durch Sprühtrocknung, gegebenenfalls in Gegenwart von Sprühhilfsmitteln, in einem Warmluftstrom oder durch Gefriertrocknung. Verfahren zur Sprühtrocknung und zur Gefriertrocknung sind dem Fachmann dem Prinzip nach bekannt und können auf die Trocknung der vorstehend beschriebenen Polymerdispersionen übertragen werden.

Bei einer Sprühtrocknung wird beispielsweise so vorgegangen, dass man die zu trocknenden Polymerisatdispersionen in einem üblichen Trockenturm in einem Warmluftstrom versprüht. Hierbei liegt die Eingangstemperatur des Warmluftstroms im Bereich von 100 bis 200 °C, vorzugsweise 120 bis 160 °C, und die Ausgangstemperatur des Warmluftstroms im Bereich von 30 bis 90 °C und vorzugsweise 60 bis 80 °C. Das Versprühen der wässrigen Polymerisatdispersion im Warmluftstrom kann beispielsweise mittels Ein- oder Mehrstoffdüsen oder über eine rotierende Scheibe erfolgen. Die Abscheidung der Polymerisatpulver erfolgt normalerweise unter Verwendung von Zyklonen oder Filterabscheidern. Die versprühte, wässrige Polymerisatdispersion und der Warmluftstrom werden vorzugsweise parallel geführt.

Als Sprühhilfsmittel, die auch als Trocknungshilfsmittel bezeichnet werden, kommen sowohl neutrale, kationische, anionische oder amphotere, wasserlösliche Polymere in Betracht. Diese haben in der Regel ein Molekulargewicht M_{N} im Bereich von 1000 bis 1000000, vorzugsweise 2000 bis 100000. Beispiele für derartige Polymere sind die zuvor als Schutzkolloide genannten Polymere.

Konkrete Beispiele für neutrale Polymere sind: Polyvinylalkohole (siehe z. B. EP-A-56 622, EP-A-680 993, DE-A-22 14 410 und DE-A-26 14 261), Polyvinylpyrrolidone (siehe z. B. DE 22 38 903 und EP 576 844). Beispiele für anionische Polymere sind Phenolsulfonsäure-Formaldehyd-Kondensate (siehe z. B. EP-A 407 889, WO 98/03576), Naphthalinsulfonsäure-Formaldehyd-Kondensate (siehe z. B. WO 98/03577), Homo- und Copolymerisate der 2-Acrylamido-2-methylpropansulfonsäure (siehe z. B. EP-A 629 650, EP-A 671 435 und DE-A 195 39 460), Copolymere ethylenisch ungesättigter Carbonsäuren, wie insbesondere Acrylsäure, Methacrylsäure und Maleinsäure, mit hydrophoben Comonomeren wie Styrol (siehe z. B. EP 467 103) oder Olefinen (siehe z. B. EP 9 169) oder mit Hydroxyalkylestern (siehe z. B. JP 59 162 161). Beispiele für kationische Polymere sind Copolymere und Terpolymere des Vinylpyrrolidons und/oder des Vinylcaprolactams mit 1-Vinyl-3-alkylimidazolinium-Salzen, z. B. mit 1-Vinyl-3-methylimidazoliniumchlorid oder -methosulfat; Copolymere und Terpolymere des Vinylpyrrolidons und/oder des Vinylcaprolactams mit (Meth)acryloyloxyethyltrialkylammoniumsalzen oder mit (Meth)acryloyloxyethylammoniumsalzen. Derartige kationische Polymere sind dem Fachmann bekannt und im Handel erhältlich.

Geeignete amphotere Polymere sind Copolymere der Acrylsäure und gegebenenfalls hydrophober Monomere, wie Styrol und gegebenenfalls wasserlöslicher, neutraler Monomere mit den unter D genannten kationischen Monomeren DK, z. B. Copolymere der Acrylsäure mit Styrol und mit (Meth)acryloxyethyltrialkylammoniumsalzen sowie gegebenenfalls mit weiteren Comonomeren, wie (Meth)acrylamid und Acetonitril. Derartige Copolymere sind beispielsweise aus der EP-A 51 144 bekannt.

Die erfindungsgemäß verwendeten wässrigen Dispersionen oder Pulver der farbstoffhaltigen Polymerisate PF können in bekannter Weise in kosmetische Mittel eingearbeitet werden. Je nach Art des kosmetischen Mittels werden wässrige Dispersionen oder Pulver bevorzugt. Die Einarbeitung in das kosmetische Mittel erfolgt nach den hierfür üblichen Vorgehensweisen, in der Regel durch Einrühren und Homogenisieren in die übrigen Bestandteile des kosmetischen Mittels. Ein Aufschließen des Pulvers ist im Unterschied zu handelsüblichen Pigmenten nicht erforderlich.

Beispiele für kosmetische Mittel, die als dekorative kosmetische Mittel ausgestaltet werden, sind Mittel zur Behandlung der Gesichtshaut, insbesondere im Augenbereich, wie Kajal-Stifte, Eyeliner-Stifte, Augenbrauenstifte, Lidschatten, Cremerouge, Puderrouge, Make-up, Schminke, z. B. Theaterschminke, Lippenstifte; Mittel zur Behandlung von Augenbrauen und Wimpern, wie Mascara und Augenwimpernschminke; Nagellacke sowohl auf Lösungsmittelbasis als auch auf Wasserbasis; Haarbehandlungsmittel, wie Haargele, z. B. Wet-Gel, Stylinggel, Haarsprays, Haarmascara, Stylingmousse, Haarschaum, Haarshampoo; weiterhin farbige Seifen; Sonnenschutzmittel, z. B. Sun-Block-Cremes und Sun-Block-Stifte. Letztere enthalten wenigstens ein Polymerisat PF, das einen der oben genannten UV-Absorber als Farbstoff F enthält. Vorzugsweise liegt der Gehalt an UV-Absorber in derartigen Polymerisaten PF im Bereich von 5 bis 30 Gew.-%, bezogen auf die Polymermatrix. Die Menge an Polymerisat PF richtet sich naturgemäß nach dem gewünschten Sonnenschutz und der im Polymerisat enthaltenen Menge an UV-Absorber.

Bei kosmetischen Mitteln auf Wasserbasis, beispielsweise wässrigen Nagellacken, Mascara, Make-ups vom Typ O/W oder Make-ups vom Typ W/O, bei dekorativen Haarpflegemitteln, wie Wet-Gel, Stylinggel, Haarspray, Haarmascara, oder Stylingmousse wird man der Einfachheit halber vorzugsweise wässrige Dispersionen der farbstoffhaltigen Polymerisate PF einsetzen. Hingegen wird man bei kosmetischen Mitteln, die ausschließlich aus Ölen oder Fetten bestehen, insbesondere solche, die eine feste Form haben, z. B. Stifte, wie Kajal-Stifte, Eyeliner-Stifte, Augenbrauenstifte, stiftförmige Theaterschminke, Lippenstifte und ähnliche, sowie bei pulver- oder puderförmigen kosmetischen Mitteln wie Lidschatten und Cremerouge oder loses Puderrouge ein pulverförmiges, farbstoffhaltiges Polymerisat PF einsetzen.

Die Menge an farbstoffhaltigem Polymerisat PF in dein kosmetischen Mittel richtet sich in erster Linie nach dem gewünschten Farbeindruck, den das dekorative kosmetische Mittel aufweisen soll. Je nach Art des kosmetischen Mittels und des gewünschten Farbeindrucks liegt der Gehalt an farbstoffhaltigem Polymerisat in dem kosmetischen Mittel im Bereich von 0,1 bis 50 Gew.-%, bezogen auf das Gesamtgewicht des kosmetischen Mittels.

Bei Nagellacken wird man beispielsweise 1 bis 10 Gew.-%, bezogen auf den Nagellack, an farbstoffhaltigem Polymerisat PF einsetzen. Bei Mascara und Augenwimpernschminken setzt man in der Regel 2 bis 20 Gew.-%, bezogen auf das kosmetische Mittel, wenigstens eines farbstoffhaltigen Polymerisats PF als farbgebenden Bestandteil ein. In Kosmetikstiften, wie Kajal-Stiften, Eyeliner-Stiften, Augenbrauen-Stiften, Eye-Shadow-Stiften wird man in der Regel 10 bis 40 Gew.-% farbstoffhaltiges Polymerisat einsetzen. Bei Lidschatten liegt der Gehalt an Polymerisat PF in der Regel noch höher und kann bis zu 50 Gew.-% betragen. Bei Cremerouge und losen Puderrouge liegt der Gehalt an farbstoffhaltigem Polymerisat PF häufig im Bereich von 1 bis 20 Gew.-%, insbesondere im Bereich 2 bis 15 Gew.-%. Bei Lippenstiften wird man häufig je nach gewünschtem Farbeindruck 2 bis 40 Gew.-%, insbesondere 5 bis 30 Gew.-% farbstoffhaltiges Polymerisat, bezogen auf das Gesamtgewicht des Lippenstiftes einsetzen. Bei Haargel und Stylinggel sowie bei Haarspray wird man in der Regel geringere Gehalte an farbstoffhaltigem Polymerisat PF einsetzen, z. B. 0,1 bis 10 Gew.-%, insbesondere 0,5 bis 5 Gew.-%.

Selbstverständlich kann man zusätzlich auch andere Pigmente des Standes der Technik einsetzen, wobei diese teilweise die farbstoffhaltigen Polymerisate PF ersetzen oder diese zur Veränderung des Farbeindrucks ergänzen können. Die Menge an zusätzlichen Pigmenten des Standes der Technik liegt bei den erfindungsgemäßen kosmetischen Mitteln in der Regel im Bereich von 0,1 bis 30 Gew.-%, bezogen auf das Gesamtgewicht des kosmetischen Mittels und richtet sich naturgemäß nach der Art des kosmetischen Mittels und nach dem gewünschten Farbeindruck.

Die erfindungsgemäß zur Anwendung kommenden, feinteiligen, farbstoffhaltigen Polymerisate PF haben gegenüber den Pigmenten des Standes der Technik zum einen den Vorteil, dass sie sich leichter in die kosmetischen Mittel einarbeiten lassen, da ein Anreiben und Aufschließen des Pigments nicht erforderlich ist. Dies gilt sowohl für die wässrigen Polymerdispersionen als auch für die Pulver des Polymerisats PF. Der mögliche Einsatz wässriger Polymerdispersionen erleichtert insbesondere auch die Herstellung solcher Formulierungen, die einen hohen Wasseranteil und einen geringen oder gar keinen Fettanteil aufweisen. Zudem weisen die farbstoffhaltigen Polymerisate PF eine höhere Farbtiefe als vergleichbare Pigmente des Standes der Technik auf. Im Unterschied zu dem Pigmenten des Standes der Technik bedarf es bei einer Veränderung des Farbtons des kosmetischen Mittels nicht einer speziellen Anpassung der übrigen Bestandteile an den neuen farbgebenden Bestandteil. Da es möglich ist, in einem Polymermatrix-Typ die unterschiedlichsten Farbstoffe einzubauen, kann man bei einmaliger Anpassung der Polymermatrix an das jeweilige kosmetische Mittel eine breite Palette von Farbmitteln zur Verfügung stellen.

Das erfindungsgemäße kosmetische Mittel kann als Suspension oder Dispersion in Lösungsmitteln oder Fettkörpern, als Emulsion, wie z. B. als Creme oder als Milch, als Pomade, Gel oder als fester Stift vorliegen; es kann als Aerosol konditioniert sein oder als Schaum vorliegen.

Das kosmetische Mittel enthält neben dem Polymerisat PF die für den jeweiligen Mittel-Typ üblichen kosmetischen Bestandteile bzw. Ingredienzien.

Beispiele für übliche kosmetische Ingredienzien sowie zahlreiche Formulierungsbeispiele für kosmetische Mittel sind in K. Schrader, Grundlagen und Rezepturen der Kosmetika, 2. Auflage, Hüthig-Buchverlag, Heidelberg, 1989, genannt.

Zu den Ingredienzien, welche prinzipiell in den kosmetischen Mitteln der Erfindung vorhanden sind, zählen Lösungsmittel, wie Wasser, niedrige Monoalkohole oder Polyole mit 1 bis 6 Kohlenstoffatomen oder Mischungen davon; die besonders bevorzugten Monoalkohole oder Polyole sind Ethanol, i-Propanol, Propylenglycol, Glycerin und Sorbit; des Weiteren sind vorhanden Fettkörper, wie mineralische, tierische, pflanzliche oder synthetische Öle oder Wachse, Fettsäuren, Fettsäureester, wie Triglyceride von C₆-C₁₂-Fettsäuren, Fettalkohole, Vaseline, Paraffin, Lanolin, hydriertes Lanolin, acetyliertes Lanolin und Siliconöl.

Das kosmetische Mittel enthält in der Regel die für den jeweiligen Mittel-Typ üblichen kosmetischen Adjuvanzien, wie Verdickungsmittel, weichmachende Mittel, Hydratisierungsprodukte, grenzflächenaktive Mittel, Konservierungsmittel, Sequestriermittel, Antioxidationsmittel, Antischaummittel, Öle, Wachse, Lanolin, Parfums, Treibmittel, Farbstoffe, Vitamine oder andere in der Kosmetik gewöhnlich verwendete Ingredienzien.

Ist das Mittel als Aerosol konditioniert, verwendet man klassische Treibmittel, wie Alkane, Distickstoffoxid und Dimethylether.

Emulsionen in Form einer Creme oder eines Make-ups enthalten außer den Polymerisaten PF Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser.

Die Konzentration des Emulgatorsystems beträgt in der Regel 4 und 35 %, bezogen auf das Gesamtgewicht der Emulsion; die Fettphase macht häufig zwischen 10 und 90 % aus und die wässrige Phase zwischen 10 und 90 %, bezogen auf das Gesamtgewicht der Emulsion. Bei den Emulgatoren handelt es sich um diejenigen, welche in diesem Emulsionstyp üblicherweise verwendet werden. Sie werden insbesondere ausgewählt unter:
- C₁₂-C₁₈-Sorbitan-Fettsäureestern,
- Estern von Hydroxystearinsäure und C₁₂-C₃₀-Fettalkoholen,
- Mono- und Diestern von C₁₂-C₁₈-Fettsäuren und Glycerin oder Polyglycerin,
- Kondensaten von Ethylenoxid und Propylenglycolen,
- oxypropylenierten/oxyethylenierten C₁₂-C₂₀-Fettalkoholen,
- polycyclischen Alkoholen, wie Sterolen,
- aliphatischen Alkoholen mit einem hohen Molekulargewicht, wie Lanolin,
- Mischungen von oxypropylenierten/polyglycerinierten Alkoholen und Magnesiumisostearat,
- Succinestern von polyoxyethylierten oder polyoxypropylenierten Fettalkoholen,
- Magnesium-, Calcium-, Lithium-, Zink- oder Aluminiumlanolat und -stearat, gegebenenfalls als Mischung mit hydriertem Lanolin, Lanolinalkohol, oder Stearinsäure oder Stearylalkohol.

Zu den Fettprodukten, aus welchen die Fettphase der Emulsionen besteht, zählen:
- Kohlenwasserstoff-Öle, wie Paraffinöl, Purcellinöl, Perhydrosqualen und Lösungen mikrokristalliner Wachse in diesen Ölen,
- tierische oder pflanzliche Öle, wie Süßmandelöl, Avocadoöl, Calophylumöl, Lanolin und Derivate davon, Ricinusöl, Pferdeöl, Schweineöl, Sesamöl, Olivenöl, Jojobaöl, Karité-Öl, Hoplostethus-Öl,
- mineralische Öle, deren Destillationsbeginn unter Atmosphärendruck bei ca. 250 °C und deren Destillationsendpunkt bei 410 °C liegt, wie z. B. Vaselinöl,
- Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinolat.

Die Fettphase kann auch in anderen Ölen lösliche Siliconöle, wie Dimethylpolysiloxan, Methylphenylpolysiloxan und das Siliconglycol-Copolymer, Fettsäuren und Fettalkohole enthalten.

Um die Retention von Ölen zu begünstigen, kann man auch Wachse verwenden, wie z. B. Carnauba-Wachs, Candellilawachs, Bienenwachs, mikrokristallines Wachs, Ozokeritwachs und Ca-, Mg- und Al-Oleate, -Myristate, -Linoleate und -Stearate.

Die Emulsionen können auch in Form eines Stiftes vorliegen. In diesem Fall beträgt die Konzentration der Wasserphase in der Emulsion im Allgemeinen 5 bis 70 Gew.-%, bezogen auf das Gesamtgewicht der Emulsion.

Fettgele enthalten in der Regel ein Öl oder ein Wachs und ein Verdickungsmittel, wie Kieselerde. Die ölig-alkoholischen oder wässrig-alkoholischen Gele enthalten einen oder mehrere niedrige Alkohole und Polyole, wie Ethanol, Propylenglycol oder Glycerin, ein Verdickungsmittel, wie Kieselerde, Cellulosederivate, Polyacrylsäurederivate und Guar-, Carouba- und Xanthangummi in Anwesenheit von Öl bzw. von Wasser.

Feste Stifte bestehen in der Regel aus Fettkörpern, wie natürlichen oder synthetischen Wachsen und Ölen, Fettalkoholen, Fettsäureestern und Lanolin.

Kosmetische Mittel auf Wasserbasis enthalten häufig Gelbildner, wie Hydrokolloide und halbfeste Fette und Wachse, z. B. Guargummi, Xanthangummi, Tragant, Alginate, Stärke, Stärkederivate, Gelatine, Cellulose und Cellulosederivate, wie Methylcellulose, Natrium-Carboxymethylcellulose, Hydroxypropylcellulose, Hydroxyethylcellulose und Polyacrylate.

Haarbehandlungsmittel, insbesondere solche auf alkoholischen oder auf Wasserbasis, z. B. Haarsprays, Haargele wie Wet-Gel oder Stylinggel, enthalten häufig auch Haarpolymere zur Festigung, Strukturverbesserung oder Formgebung.

Beispiele für Haarpolymere sind insbesondere:
- nicht-ionische, wasserlösliche bzw. wasserdispergierbare Polymere oder Oligomere, wie Polyvinylcaprolactam, z. B. Luviskol Plus (BASF), oder Polyvinylpyrrolidon und deren Copolymere, insbesondere mit Vinylestern, wie Vinylacetat, z. B. Luviskol VA 37 (BASF); Polyamide, z. B. auf Basis von Itaconsäure und aliphatischen Diaminen, wie sie z. B. in der DE-A-43 33 238 beschrieben sind;
- amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®);
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer. Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Methacrylsäure), Natriumsulfonat-haltige Polyamide oder Natriumsulfonat-haltige Polyester, oder
- kationische (quaternisierte) Polymere, z. B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z. B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die erfindungsgemäßen kosmetischen Zusammensetzungen werden hergestellt, in dem man das farbstoffhaltige Polymerisat PF, enwerder als Pulver oder als wässrige Dispersion, in eine in der Regel farblose Basismischung aus kosmetischen Bestandteilen, die für die jeweilige Zusammensetzung typisch sind, einarbeitet. Das Einarbeiten gelingt in der Regel durch einfaches Vermischen des Polymerisats PD mit wenigstens einem Bestandteil der kosmetischen Zusammensetzung, oder einer Mischung von Bestandteilen der kosmetischen Zusammensetzung z. B. in Form einer O/W- oder einer W/O-Emulsion, einer Schmelze, eines Puders, eines Gels, einer Lösung, einer Fett- oder einer Wasserphase. Üblicherweise wird man die erhaltene Mischung anschließend in den hierfür üblichen Apparaturen homogenisieren. Das Polymerisat PF kann sowohl als Pulver als auch als wässrige Dispersion eingearbeitet werden, je nach dem ob es sich bei den kosmetischen Bestandteilen um feste Bestandteile, um hydrophobe oder um hydrophile flüssige Bestandteile handelt. Die erhaltene Mischung wird anschließend nach bekannten Verfahren in ihre gebrauchsfertige Form überführt. Verfahren zur Herstellung kosmetischer Zusammensetzungen sind aus dem Stand der Technik bekannt, z. B. von K.Schrader, Grundlagen und Rezepturen der Kosmetika 2. Auflage, Hüthig-Buchverlag, Heidelberg, 1989 p. 905 to 935.

Die im Folgenden angegebenen Beispiele für farbstoffhaltige Polymerisate PF in Form wässriger Dispersionen oder Pulver sowie die kosmetischen Formulierungsbeispiele sollen die vorliegende Erfindung näher erläutern.

### Beispiele für erfindungsgemäß geeignete Polymerisate PF

### I. Allgemeines

Die Bestimmung der Polymerisatteilchengröße (d_{z}-Wert) erfolgte in der oben beschriebenen Weise mittels eines Coulter N4 Plus Particle Analyzers an 0,01 gew.-%igen Proben der Dispersion.

Die relative Lichtdurchlässigkeit (LD-Wert) für weißes Licht wurde an einer 0,01 gew.-%igen Probe bei einer Schichtdicke von 2,5 cm gegen Wasser bestimmt.

Die Viskosität wurde in einem Eprecht-Viskosimeter A-III bei 23 °C für 20 min bestimmt.

### Eingesetzte Farbstoffe

Farbstoff 1: Lumogen® F Gelb 083:1,7-Bis(isobutyloxycarbonyl)-6,12-dicyanoperylen
Farbstoff 2: Lumogen® F Rot 300 (BASF; Perylentetracarbonsäurediimid-Fluoreszenzfarbstoff);
Farbstoff 3: C.I. Solvent Yellow 162;
Farbstoff 4: Neopen® Cyan 742 (BASF; Azamethinfarbstoff)
Farbstoff 5: Neopen® Magenta 525 (BASF; Azamethinfarbstoff)
Farbstoff 6: Neopen® Blau FF 4012 (BASF; Cu-Phthalocyaninfarbstoff);
Farbstoff 7: C.I. Solvent Red 119
Farbstoff 8: C.I. Basic Red 14 + Dodecylsulfonat (1:1)*
Farbstoff 9: Rhodamin 2C Base + Ölsäure (1:1)*

- UV-Absorber 1:: 4-Methoxyzimtsäure-n-octylester
- UV-Absorber 2:: 4,4-Diphenylbutadien-1,1-dicarbonsäure- bis(2-ethylhexyl)ester,
- UV-Absorber 3:: 4-tert.-Butyl-4'-methoxydibenzoylmethan,
- UV-Absorber 4:: 2,4,6-Tris-{N-[4-(2-ethylhex-1-yl)oxycarbonylphenyl]amino}-1,3,5-triazin,
- UV-Absorber 5:: 2-Cyano-3,3-diphenylacrylsäure-(2'-ethylhexyl)ester (Octocrylen)

* Komponenten wurden im Molverhältnis 1:1 eingesetzt

Es werden die folgenden Abkürzungen verwendet:
- AS:: Acrylsäure
- BDDA:: Butandioldiacrylat
- DVB:: Divinylbenzol
- HD:: Hexadecan
- L370:: Luviquat® FC 370
- MAS:: Methacrylsäure
- MMA:: Methylmethacrylat
- NaPS:: Natriumperoxodisulfat
- NLS:: Natriumlaurylsulfat (15 gew.-%ig)
- CP:: Copolymer aus 68 Gew.-% Styrol, 10 Gew.-% Acrylsäure und 22 Gew.-% N-(3-Dimethylaminopropyl)methacrylamid (20 gew.-%ig in Eisessig/Wasser 1:6)
- S:: Styrol
- SA:: Stearylacrylat
- T:: Tamol® NN4501

Luviquat® FC 370 ist eine etwa 40 gew.-%ige wässrige Lösung eines Copolymerisats aus Vinylpyrrolidon und 1-Vinyl-3-methylimidzoliumchlorid im Gew.-verhältnis 7:3, mit einem K-Wert (nach Fikentscher; bestimmt in Anlehnung an DIN 53726 als 1 gew.-%iger Lösung in 3 gew.-%iger wässriger NaCl-Lösung) im Bereich von 41 bis 49 (Hersteller: BASF AG, Ludwigshafen).

Tamol® NN 4501 ist das pulverförmige Natriumsalz eines niedermolekularen Formaldehydkondensationsprodukts eines Isomergemischs aus α- und β-Naphthalinsulfonsäure, dessen 45 gew.-%ige, wässrige Lösung einer mittleren Viskosität (nach Brookfield) von etwa 70 mPa·s (bestimmt bei 23 °C) aufweist (Hersteller: BASF AG, Ludwigshafen).

### II. Herstellung wässriger Dispersionen farbstoffhaltiger Polymerisate (PF Nr. 1 bis 10):

### 1. Herstellung durch Batchverfahren (allgemeine Vorschrift in Anlehnung an WO 99/40123 V 1., Polymerisate PF Nr. 6 bis 10):

### 1.1 Herstellung der farbstoffhaltigen Miniemulsion

In einem Reaktionsgefäß mit Rührer legte man 250 g entionisiertes Wasser und 25 g einer 5 gew.-%igen Lösung eines Copolymeren CP als Schutzkolloid vor. Bei Verwendung von H₂O₂ als Initiator legte man zusätzlich 0,5 g Ascorbinsäure und 0,5 g einer 1 gew.-%igen wässrigen Fe-EDTA-Lösung vor (Vorlage). Hierzu gab man innerhalb 2 Minuten eine Lösung des jeweiligen Farbstoffs in den zu polymerisierenden Monomeren (Monomer/Farbstofflösung). Die Bestandteile dieser Lösung sind in Tabelle 1 angegeben. Bei Verwendung von tert-Butylhydroperoxid als Initiator wurde dieser nach Lösen des Farbstoffs in den Monomeren gelöst. Anschließend rührte man weitere 10 Minuten. Die dabei resultierenden, konventionellen, farbstoffhaltigen Monomeremulsionen wurden anschließend wie folgt zu einer wässrigen Monomermikroemulsion mittels Ultraschall homogenisiert:

Als Ultraschallquelle diente die in Figur 4 der DE 197 56 874 beschriebene Vorrichtung, ausgerüstet mit einer Durchflusszelle mit einem Gefäßdurchmesser von 42 mm und einer Höhe von 25 mm. Die Sonotrode hatte einen Durchmesser von 40 mm und eine Leistung von 1000 W. Unter Rühren wurde jeweils ca. 0,5 Liter der wässrigen Makroemulsion mit einer Durchflussgeschwindigkeit von 30 l/h mit einer Leistung von 1000 W beschallt. Hierbei wurden wässrige, farbstoffhaltige Mini-Emulsionen erhalten.

### 1.2 Polymerisation der farbstoffhaltigen Miniemulsion

Die so erhaltene Miniemulsion wurde in einem Polymerisationsgefäß vorgelegt und auf 80 °C erwärmt. Dann gab man unter Rühren in einer Portion die Initiatorlösung zu, ließ 2,5 h bei 80 bis 85 °C nachreagieren und kühlte dann auf 25 °C ab. Man erhielt eine etwa 30 bzw. 35 gew.-%ige wässrige Dispersion des Polymerisats PF. Die mittleren Teilchengrößen der so erhaltenen Polymerdispersionen sind in Tabelle 1 angegeben.

Initiatorlösung: 0,7 g H₂0₂ in 28 g Wasser

### 2. Herstellung durch Zulaufverfahren (Polymerisat PF Nr. 1-5):

Zunächst wurde in der unter II 1.1 beschriebenen Weise aus 170 g Wasser, 2 g einer 15 gew.-%igen Natriumlaurylsulfat-Lösung und einer Lösung von 10 g Farbstoff 2 und 1 g t-BPO in 100 g MMA und 5 g BDDA eine Miniemulsion hergestellt (Zulauf 1).

29 g der so erhaltenen Mini-Emulsion legte man zusammen mit 0,3 g einer 6 gew.-%igen wässrigen Ascorbinsäurelösung (Zulauf 2) und 0,3 g einer 1 gew.-%igen Fe-EDTA-Lösung vor. Man erwärmte auf 80 °C und gab dann, zeitgleich beginnend innerhalb 1 h unter Beibehaltung der 80 °C die farbstoffhaltige Mini-Emulsion und 5,3 g von Zulauf 2 in das Polymerisationsgefäß und ließ nach beendeter Zugabe 1 h nachpolymerisieren. Der Feststoffgehalt der Dispersion lag bei 39 Gew.-%. Die mittlere Teilchengröße lag bei 200 nm.

In ähnlicher Weise wurden die farbstoffhaltigen Polymerisate PF Nr. 2 bis Nr. 5 hergestellt.

Als Initiator diente eine Lösung von 1 g Natriumperoxodisulfat in 39 g Wasser. Die Vorlage enthielt 4 g der Initiatorlösung und 100 g Wasser.

Als Emulgator wurde, sofern nichts anderes angegeben ist, der Bis-2-Ethylhexylester der Sulfobernsteinsäure (als Natriumsalz in Form einer 60-gew.-%igen, wässrigen Lösung) verwendet (LUMITEN® IRA der BASF AG) und zwar 0,6 g bei der Herstellung von Polymerisat PF Nr.2 und 1,2 g bei Polymerisat PF Nr. 3, 4 und 5. Bei der Herstellung von Polymerisat PF Nr. 3 setzte man zusätzlich 4,8 g Tamol NN4501 ein, bei der Herstellung von PF Nr. 5 zusätzlich 4,8 g Luviquat FC 370.

Die Teilchengrößen der Polymerisate PF sind in Tabelle 1 angegeben.

Alle Polymerisate PF ließen sich unter Kühlung im Trockeneis/Acetonbad durch Anlegen von Vakuum zu redispergierbaren Pulvern gefriertrocknen.

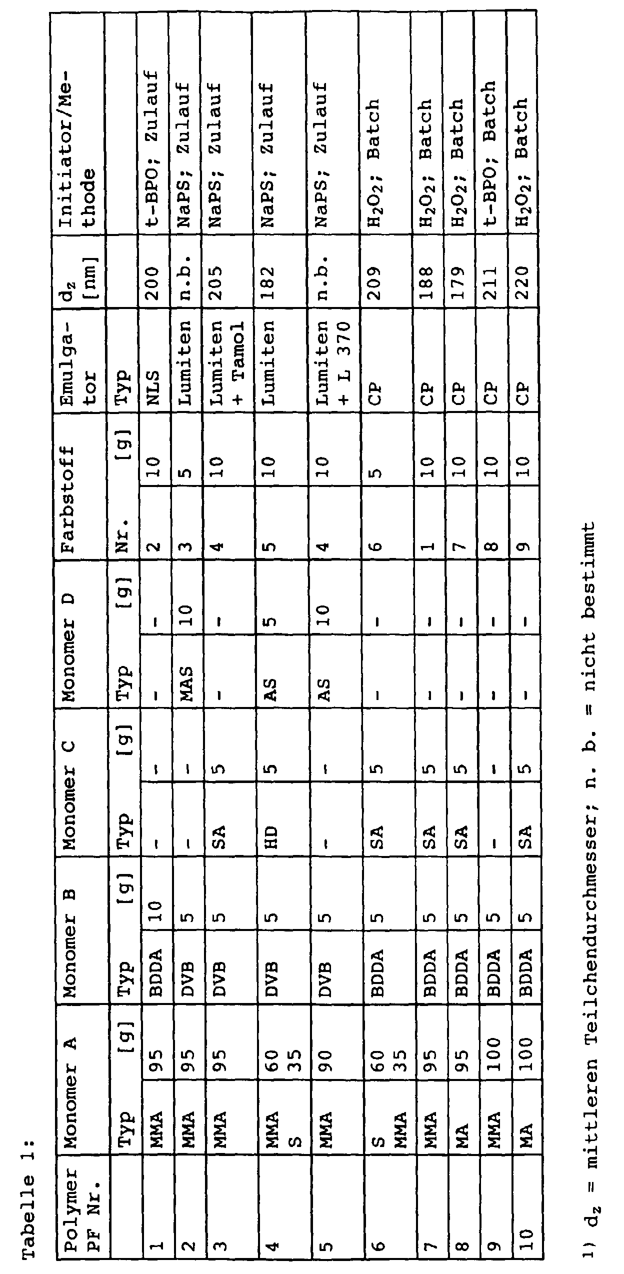

### III.Herstellung wässriger Dispersionen UV-Absorber-haltiger Polymerisate (PF Nr. 11 bis 19):

### 1. Herstellung der Miniemulsion (allgemeine Vorschrift)

In einem Reaktionsgefäß mit Rührer legte man entionisiertes Wasser und 0,6 g Natriumdodecylsulfat vor. Die Gesamtmenge an Wasser betrug etwa 450 ml. Hierzu gab man innerhalb 2 Minuten eine Lösung des jeweiligen UV-Absorbers in den zu polymerisierenden Monomeren (Monomer/Farbmittellösung). Die Einsatzmengen und Art des UV-Absorbers sind in Tabelle 2 angegeben. Die Zusammensetzung der Monomerlösung war in allen Beispielen wie folgt: 5 g Stearylacrylat, 5 g Butandioldiacralat, 95 g Methylmethacrylat. Anschließend rührte man weitere 10 Minuten. Die dabei resultierenden, konventionellen, farbstoffhaltigen Monomeremulsionen wurden anschließend wie folgt zu einer wässrigen Monomerminiemulsion mittels Ultraschall homogenisiert.

Als Ultraschallquelle diente ein Branson Sonifier II 450. Die Ultraschallbehandlung erfolgte unter Rühren der Emulsion durch 5-minütige Beschallung bei der Einstellung duty-cycle 25%, output 10, und 10 minütige Beschallung bei der Einstellung duty cycle 100% und output 10. Die Tröpfchengröße (Volumenmittel) in der Monomeremulsion lag unterhalb 400 nm.

Die nach 1. erhaltene Miniemulsion wurde in einem Polymerisationsgefäß vorgelegt und auf 80 °C erwärmt. Dann gab man unter Rühren in einer Portion die Initiatorlösung (1 g Natriumperoxodisulfat in 38 ml Wasser bzw. 0,3 g H₂O₂ als 35 gew.-%ige wässrige Lösung und 0,3 g Ascorbinsäure als wässrige Lösung bei PF 19) zu, ließ 3,5 h bei 80 bis 85 °C nachreagieren, kühlte dann auf 25 °C ab und filtrierte zur Bestimmung des Koagulatanteils über ein 75 µm Sieb. Man erhielt eine etwa 20 gew.-%ige wässrige Dispersion des Polymerisats PF. Der Anteil der Koagulatbildung war in allen Fällen gering (< 5 %, bezogen auf die Einsatzstoffe).

Die Lichtdurchlässigkeit (LD-Wert), der pH-Wert, die Viskosität und die Lagerstabilität der so erhaltenen Polymerdispersionen sind in Tabelle 1 angegeben. Die mittlere Teilchengröße der Polymerisatteilchen lag herstellungsbedingt unterhalb 400 nm.

Die Dispersionen waren lagerstabil und ließen sich zu einem Pulver gefriertrocknen.

**Tabelle 2**

| PF | UV-Absorber | | LD ¹⁾ [%] | pH | Viskosität [mPas] |
|---|---|---|---|---|---|
| | Typ | [Gew.-%] | | | |
| 11 | UV2 | 5 | 88,1 | 2,1 | 3,4 |
| 12 | UV2 | 20 | 81,0 | 2,2 | 3,7 |
| 13 | UV5 | 20 | 82,0 | 2,2 | 3,7 |
| 14 | UV1 | 20 | 80,7 | 2,2 | 3,7 |
| 15 | UV3 | 20 | 82,3 | 2,0 | 3,7 |
| 16 | UV4 | 20 | 84,0 | 2,6 | 3,7 |
| 17 | UV1 | 30 | 77,4 | 2,2 | 3,7 |
| 18 | UV1 | 40 | 76,3 | 2,3 | 3,7 |
| 19 | UV5 | 20 | 79,4 | 2,6 | 3,9 |

| | | | | | |
|---|---|---|---|---|---|
| 1) Lichtdurchlässigkeit | | | | | |

Untersuchung des Sonnenschutzfaktors SPF:

Die Bestimmung des Sonnenschutzfaktors erfolgte mittels eines SPF-Analysers an Proben, die 3 Gew.-% UV-Absorber enthielten.
- Probe 1:: 3 g UV-Absorber UV1 und 1 g Cremophor RH60 in 96 g Wasser (Vergleich)
- Probe 2:: 3 g UV-Absorber UV5 und 1 g Cremophor RH60 in 96 g Wasser (Vergleich)
- Probe 3:: 15 g Polymerisat PF14 in 85 g Wasser (entspricht 3 % UV1)
- Probe 4:: 15 g Polymerisat PF13 in 85 g Wasser (entspricht 3 % UV5)
- Probe 5:: 15 g Polymerisat PF14 in 85 g Butylenglykol (entspricht 3% UV1)
- Probe 6:: 15 g Polymerisat PF13 in 85 g Butylenglykol (entspricht 3% UV5)

Die Ergebnisse der Untersuchung sind in Tabelle 3 angegeben:

**Tabelle 3**

| SPF-Werte | | |
|---|---|---|
| Probe | UV-Absorber | SPF-Wert |
| 1 | UV1 | 9,1 |
| 2 | UV2 | 6,4 |
| 3 | UV1 | 9,0 |
| 4 | UV2 | 9,1 |
| 5 | UV1 | 10,3 |
| 6 | UV2 | 10,7 |

Im Folgenden sind beispielhaft Formulierungen von Polymerisaten PF in kosmetischen Pflegemitteln beschrieben. Alle Angaben sind in Gramm. Die Mengenangaben der wässrigen Dispersionen des farbstoffhaltigen Polymerisats PF sind auf den Polymerisatanteil bezogen.

### Nagellackformulierungen (Formulierung 1 bis 3)

### Formulierung 1:

- 26,3: Nitrocellulose
- 4,9: Copolymer aus Polyoxyisobutylen und Methylenharnstoff
- 7,8: Copolymer aus Butylacrylat und Vinylisobutylether, 50 Gew.-% in Essigester (Acronal®700 L 50 % BASF)
- 4,9: Methoxypropylacetat
- 53,5: Butylacetat
- 2,6: Polymerisat PF als Pulver

### Formulierung 2:

- 16,0: Nitrocellulose
- 4,0: Toluolsulfonamid-Formaldehyd-Harz
- 5,0: Dibutylphthalat
- 10,0: Butylacetat
- 10,0: Ethylacetat
- 10,0: Ethanol
- 40,0: Toluol
- 5,0: Polymerisat PF als Pulver

Die Bestandteile mit Ausnahme der wässrigen Dispersion von PF werden gelöst. Dann wird das farbstoffhaltige Polymerisat PF als wässrige Dispersion eingerührt und die Mischung anschließend homogenisiert.

### Wässriger Nagellack: (Formulierungen 3 bis 4)

### Formulierung 3:

- 27,2: Wässrige Polyurethan-Dispersion
- 13,8: Acrylsäure-Styrol-Copolymer
- 0,08: Polyacrylsäure-Verdicker
- 0,5: Butylglykolacetat
- 2,4: Polymerisat PF als wässrige Dispersion Wasser ad 100

Das Polyurethan wird als feindisperse wässrige Dispersion vorgelegt. Das Acrylsäure-Styrol-Copolymer wird als wässrige Dispersion unter Rühren hinzugefügt. Anschließend gibt man unter Rühren den Acrylatverdicker zu. Es wird weiter gerührt, bis die Masse hochviskos ist. Schließlich rührt man die wässrige Dispersion von PF ein.

### Formulierung 4:

Wie Formulierung 3, jedoch mit 0,4 g Acid Blue 74 Aluminium Lake und 2,0 g Polymerisat PF als wässrige Dispersion.

### Mascara

### Formulierung 5:

- 14,0: demin. Wasser
- 0,2: Antioxidans (Oxynex 2004 der E. Merck, Darmstadt)
- 2,5: Polyoxyethylen/Polyoxypropylen-Blockcopolymer (Poloxamer 407 der BASF Aktiengesellschaft)
- 3,5: Polyvinylpyrrolidon
- 11,0: Ethanol
- 0,7: Triethanolamin
- 0,52: Polyacrylsäure (CTFA: Carbomer)
- 57,58: demin. Wasser
- 10,0: Polymerisat PF als wässrige Dispersion

Man lässt die Polyacrylsäure in Wasser quellen und arbeitet dann unter Rühren die klar gelösten restlichen Bestandteile zu einem Gel. Anschließend arbeitet man die wässrige Dispersion von PF ein.

### Augenwimpernschminke

### Formulierung 6:

- 80,8: Kastoröl
- 6,0: Capryl/Caprin-Triglycerid
- 0,2: Antioxidans (Oxynex 2004 der E. Merck, Darmstadt)
- 2,0: Trihydroxystearin
- 0,3: Polyvinylpyrrolidon
- 2,0: Sorbitanoleat
- 8,7: Polymerisat PF als Pulver

Die Fettbestandteile werden ineinander gelöst. Anschließend rührt man Polyvinylpyrrolidon ein. Dann wird das pulverförmige Polymerisat PF untergemischt.

### Creme Mascara

### Formulierung 7:

- 75,0: Petroleum-Destillat
- 8,3: Quaternium-18-Hectorit
- 2,5: Propylencarbonat
- 11,5: wässrige Dispersion von PF
- 1,0: Ultramarin
- 1,7: Vinylpyrrolidon/Vinylacetat-Copolymer

Die Komponenten der Fettphase werden mittels starker Scherkräfte zu einem Gel verarbeitet. Anschließend arbeitet man die wässrige Dispersion des farbstoffhaltigen Polymerisats PF und das Vinylpyrrolidon/Vinylacetat-Copolymer ein und homogenisiert.

### Kajalstift-Kosmetikstift

### Formulierung 8:

- 34,3: Hydroxyliertes Lanolin
- 17,10: Hydriertes Coco-Glycerid
- 2,9: Lanolin
- 28,6: Glycerylstearate
- 17,1: Polymerisat PF als Pulver

Die Fettkomponenten werden bei 80 °C aufgeschmolzen. Anschließend wird das pulverförmige Polymerisat PF untergemischt, gegebenenfalls parfümiert, durch Gießen oder Extrudieren zu Minen für Kosmetikstifte geformt.

### Eye-Liner-Stift

### Formulierung 9:

- 30,0: Cyclomethicone
- 6,7: Lanolinöl
- 8,0: Carnauba Wax
- 3,3: Bienenwachs
- 22,7: Paraffinöl
- 2,7: Cetylalkohol
- 20,0: Polymerisat PF als Pulver
- 5,6: Pigment Blue 15
- 1,0: Eisenoxidpigment

### Augenbrauenstift

### Formulierung 10:

- 78,0: Cutina LM (Lippenstiftmasse der Firma Henkel KGaA,Düsseldorf)
- 12,0: Ozokerite
- 9,0: Polymerisat PF als Pulver
- 1,0: Eisenoxidpigment

### Lidschatten

### Formulierung 11

- 20: Talkum
- 10: Kartoffelstärke
- 5: Magnesiumstearat
- 45: Polymerisat PF als Pulver
- 5: Ultramarin (Sicomet Blau P 77007)
- 15: Lidschatten Binder

### Lidschatten Binder

- 35: Lanolin
- 30: Isopropylstearat
- 30: Paraffinöl
- 3: Parfümöl
- 1: Carnauba Wax
- 1: Propylparaben

Die Lidschattenbestandteile werden homogen gemischt, und das pulverförmige Polymerisat PF und das Farbpigment (Ultramarin) eingerührt. Die Binderbestandteile werden bei 70 °C geschmolzen. Die Lidschattenbestandteile werden mit dem geschmolzenen und gut vermengten Binder besprüht. Danach wird bei einem Pressdruck von 40 bis 60 bar gepresst. Man erhält einen Lidschattenpuder mit weichem Hautfeeling und einzigartigem Farbeffekt.

### Formulierung 12:

Wie vorherige Formulierung, aber mit 50 g Pulver von PF statt der Ultramarin/PF-Mischung.

### Lidschatten in Stiftform

### Formulierung 13:

- 15,0: Triglycerid einer C₁₈₋₃₆-Säure
- 5,0: Glycerylbehenat
- 35,0: Mineralisches Öl
- 15,0: Mineralisches Öl (und) Lanolin Alkohol
- 0,2: Parfümöl
- 0,8: Polyvinylpyrrolidon
- 1,5: Talkum
- 27,5: Polymer PF als Pulver

Die Fettkomponenten werden bei 80 °C geschmolzen und das pulverförmige Polymerisat PF untergemischt. Anschließend wird parfümiert und die Masse durch Gießen oder Extrudieren zu Minen für Kosmetikstifte geformt.

### Eye-Shadow-Stift

### Formulierung 14:

- 6,0: Bienenwachs
- 5,0: Carnauba Wax
- 10,0: Candelilla Wax
- 34,0: Hexyllaurat
- 20,0: Kastoröl
- 20,0: Polymer PF als Pulver
- 4,0: Chromoxid-Grün-Pigment
- 1,0: Parfümöl

Lidschattenstifte aus den beiden obigen Formulierungen können auch anstelle von reinem pulverförmigem Polymerisat PF mit Mischungen von Farbpigmenten und pulverförmigen Polymerisaten PF formuliert werden.

### Cremerouge (Formulierungen 15 und 16)

### Formulierung 15:

- 5,5: Candelilla Wax
- 8,5: Bienenwachs
- 3,0: Cetylpalmitat
- 8,5: Paraffinöl
- 43,0: Cetearyloctanoat
- 3,0: Hydrierte Kokosfettsäureglyceride
- 11,0: Vaseline
- 14,5: Talkum
- 3,0: Polymerisat PF als Pulver

Die Bestandteile der Grundmasse werden auf etwa 80 °C erhitzt und gut vermischt. Anschließend wird das pulverförmige Polymerisat PF in die Grundmischung eingearbeitet.

### Formulierung 16:

Wie Formulierung 17, jedoch statt des reinen Pulvers PF werden 0,5 g Pigment Red 57:1 und 2,5 g Pulver PF eingearbeitet.

### Loses Puderrouge (Formulierungen 17 bis 19)

### Formulierung 17:

- 77,0: Talkum
- 10,0: Magnesiumstearat
- 2,0: Calciumcarbonat
- 0,5: Vaseline
- 0,5: Paraffinöl
- 10,0: Polymerisat PF als Pulver

Die trockenen Puderbestandteile werden homogen gemischt und mit den geschmolzenen und gut vermengten Fettbestandteilen gemischt.

### Formulierung 18:

Wie Formulierung 17, jedoch kann für intensivere Rotfärbung das reine pulverförmige Polymerisat PF durch eine Mischung von 1 bis 2 g Rotpigment, z. B. Pigment Red 172 Aluminium Lake und 8 bis 9 g pulverförmiges PF ersetzt werden.

### Formulierung 19:

Wie Formulierung 18, jedoch mit 9,5 g pulverförmigem Polymerisat PF und 0,5 g Eisenoxid-Pigment.

### Make up Typ W/O

### Formulierung 20:

- 5,5: Hydriertes Kastoröl, ethoxyliert mit 7 EO-Einheiten
- 7,0: Cetearyloctanoat
- 4,5: Isopropylmyristat
- 14,0: Paraffinöl
- 0,3: Magnesiumstearat
- 0,3: Aluminiumstearat
- 2,0: PEG-45/Dodecylglykol-Copolymer
- 0,2: Propylparaben
- 5,0: Propylenglykol
- 0,6: Magnesium Sulfate
- 0,1: Paraben
- 50,8: Wasser
- 0,2: Parfümöl
- 0,5: Vitamin E-Acetat
- 9,0: wässrige Dispersion von PF (gerechnet als Feststoff)

Die Bestandteile der Fettphase und der Wasserphase erhitzt man separat auf etwa 75 °C und bringt dann die Wasserphase unter Rühren langsam in die Fettphase ein. Nach Homogenisieren kühlt man unter Rühren auf 40 °C ab, gibt Parfümöl und Wirkstoffe zu, und homogenisiert nochmals. Dann wird die wässrige Dispersion von PF untergerührt.

### Formulierung 21:

Wie Formulierung 20, jedoch mit 8 g Polymerisat PF, 0,5 g Eisenoxid-Pigment und 0,5 g Titandioxid-Pigment.

### Make up Typ O/W

### Formulierung 22:

- 1,7: Glycerylstearat
- 1,7: Cetylalkohol
- 1,7: Ceteareth-6, Stearylalkohol
- 1,7: Ceteareth-25
- 5,2: Capryl/Caprin-Triglycerid
- 0,2: Methyldibromoglutaronitril (und/oder) Phenoxyethanol
- 0,3: Imidazolidinyl-Harnstoff
- 4,3: Propylenglykol
- 69,0: Demineralisiertes Wasser
- 0,2: Parfümöl
- 14,0: Polymerisat PF als wässrige Dispersion

Die Bestandteile der Fettphase und der Wasserphase werden separat auf etwa 75 °C erhitzt. Dann bringt man die Wasserphase zusammen mit der wässrigen Dispersion von PF unter Rühren langsam in die Fettphase ein. Man homogenisiert und kühlt unter Rühren auf 40 °C ab und gibt nach Belieben Parfümöl zu und homogenisiert nochmals.

### Formulierung 23:

Wie vorheriges Formulierung, jedoch mit 12 % wässrige Dispersion von PF, 1,5 % Iron oxides und 0,5 % Titanium dioxide.

### Theaterschminke

### Formulierung 24:

- 75,0: Petroleum Destillat
- 8,3: Quaternium-18-Hectorit
- 2,5: Propylencarbonat
- 1,7: Polyvinylpyrrolidon/Vinylacetat-Copolymer
- 12,5: Polymerisat PF als Pulver

Aus den Bestandteilen wird unter Aufbietung starker Scherkräfte ein Gel hergestellt. Das Copolymer und pulverförmige Polymerisat PF wird eingearbeitet. Anschließend wird homogenisiert.

### Formulierung 25:

Wie vorheriges Formulierung 24, jedoch mit 11 g Polymerisat PF und 1,5 g konventionellem Farbpigment, z. B. Pigment Blue 15.

### Formulierung 26:

- 67,5: Mineralisches Öl
- 20,0: Bienenwachs
- 10,0: Ceresin Wachs
- 2,5: Polymerisat PF als Pulver

Fettkomponenten werden geschmolzen und mit pulverförmigen Polymerisat PF zu einer homogenen Paste verarbeitet.

### Fettschminke für das Theater in Stiftform

### Formulierung 27:

- 22,0: Ceresin Wachs
- 18,0: Bienenwachs
- 44,0: Mineralisches Öl
- 5,0: Terpentin
- 1,0: Parfümöl
- 8,0: Polymerisat PF als Pulver
- 2,0: Eisenhexacyanoferrat

Die Fettkomponenten werden bei 80 °C geschmolzen und das pulverförmige Polymerisat PF untergemischt. Anschließend wird die Masse parfümiert und durch Gießen oder Extrudieren zu Minen für Kosmetikstifte geformt.

### Lippenstift (Formulierungen 28 bis 31)

### Formulierung 28:

- 3,0: Carnauba Wax
- 3,5: Candelilla Wax
- 2,0: Bienenwachs
- 7,0: Mikrokristallines Wachs
- 1,5: Cetylpalmitat
- 5,0: Vaseline
- 3,5: Lanolin-Wachs
- 2,0: Lanolin
- 9,0: Cetearyloctanoat
- 0,2: Bisabolol
- 0,5: Tocopherol
- 2,0: Tocopheryl-Acetat
- 3,5: Hydrierte Kokosfettsäureglyceride
- 42,3: Kastoröl
- 15,0: Polymerisat PF als Pulver

Die Bestandteile der Fettmasse werden geschmolzen. Dann arbeitet man das pulverförmige Polymerisat PF in die Grundmasse ein und gießt die homogene Schmelze in auf 60 °C vorgewärmte Gießformen. Die Gießlinge werden den Formen kalt entnommen und nach Erwärmen auf Raumtemperatur noch kurz abgeflammt.

### Formulierung 29:

- 14,0: Oleylalkohol
- 10,0: Kastoröl
- 6,0: Diisopropyladipat
- 5,0: Stearamide MEA
- 10,0: Polymerisat PF als Pulver
- 1,0: Eisenoxid-Pigment
- 9,0: Stearylheptanoat
- 7,0: Isopropyllanolat
- 8,0: Carnauba Wax
- 10,0: Bienenwachs
- 5,0: Cetylalkohol
- 5,0: Ozokerite
- 3,0: Microkristallines Wachs
- 2,0: Polyethylen
- 2,0: Petrolatum
- 2,0: Mineralisches Öl
- 1,0: Parfümöl

### Formulierung 30:

- 10,0: Hydroxyoctacosanylhydroxystearat
- 9,0: Candelilla Wax
- 25,0: Kastoröl
- 7,9: Isopropylmyristat
- 5,0: Sorbitantrioleat
- 3,0: Hydroxyliertes Lanolin
- 6,0: Butylenglykol
- 0,1: Propylparaben
- 1,0: Parfümöl
- 3,0: Ultramarin
- 30,0: Polymerisat PF als Pulver

### Formulierung 31:

- 40,0: Kastoröl
- 10,0: Mineralisches öl
- 9,0: Hydriertes Kastoröl
- 5,0: Kakaobutter
- 10,0: Carnauba-Wax
- 5,0: Stearylheptanoat
- 5,0: Bienenwachs
- 10,0: Lanolin
- 5,0: Polymerisat PF als Pulver
- 1,0: Parfümöl

### Haargel-Formulierungen (Formulierungen 32 bis 34)

### Formulierung 32:

- 59,8: Wasser
- 0,5: Polyacrylsäure (CTFA: Carbomer)
- 1,2: Triethanolamin
- 29,9: Glycerin
- 2,0: Propylenglycol
- 2,3: Dimethicone-Copolyol
- 0,3: Imidazolidinyl-Harnstoff
- 4,0: Polymerisat PF als wässrige Dispersion

### Formulierung 33:

- 0,7: Polyacrylsäure (CTFA: Carbomer)
- 92,1: Wasser
- 0,7: Hydriertes Kastoröl, ethoxyliert mit 40 EO Einheiten
- 0,2: Parfümöl
- 0,3: Imidazolidinylharnstoff
- 1,0: Panthenol
- 3,0: Polyvinylpyrrolidon
- 1,0: Triethanolamin
- 1,0: Polymerisat PF als wässrige Dispersion

### Formulierung 34 (Styling-Gel):

- 0,5: Polyacrylsäure (CTFA: Carbomer)
- 74,7: Wasser
- 15,0: Ethanol
- 0,2: Hydroxyethylcetyldimonium-Phosphat
- 6,0: Polyvinylpyrrolidon
- 0,3: Imidazolidinylharnstoff
- 0,8: Tetrahydroxypropylethylendiamin
- 2,: 5 Polymerisat PF als wässrige Dispersion

### Haarsprays (Formulierungen 35 bis 37)

### Formulierung 35:

- 3,0: Polyvinylpyrrolidon
- 4,0: Vinylpyrrolidon/Vinylacetat-Copolymer
- 0,7: Rosin Acrylat
- 44,3: Ethanol
- 3,0: Polymerisat PF als wässrige Dispersion
- 45,0: Propan/Butan

Die Komponenten werden mit Ausnahme der wässrigen Dispersion des Polymeren PF gelöst. Anschließend wird die wässrige Dispersion von PF eingerührt. Vor dem Abfüllen einige Glaskugeln zugeben.

### Formulierung 36:

- 1,5: Acrylsäure/Acrylamid-Copolymer
- 0,11: Aminomethylpropanol
- 0,02: Cyclomethicone
- 6,0: Wasser
- 3,0: Polymerisat PF als wässrige Dispersion
- 60,0: Dimethylether
- 29,37: Ethanol

### Formulierung 37:

Die Formulierung entspricht Formulierung 36, jedoch werden 2 g wässrige Dispersion von PF und 1 g Pigment Blue 15 eingearbeitet.

### Haarmascara (Formulierungen 38 bis 40)

### Formulierung 38:

- 15,0: Mischung aus Bienenwachs, Carnauba (Copernicia Cerifera) Wax, Stearinsäure, Ceteareth-25, PEG-2 Stearate SE, mineralischem Öl, hydriertem Kokosöl und Cetylalcohol (Base RW 135, Wacker)
- 1,5: Dimethicone
- 0,5: Konservierungsmittel
- 42,1: Wasser
- 0,45: Triethanolamin
- 0,45: Xanthan, Hectorit und Cellulosegummi
- 30,0: Acrylsäure-Copolymer
- 10.0: Polymerisat PF als wässrige Dispersion

### Formulierung 39:

Wie Formulierung 38, aber mit 8 g Polymerisat PF und 2 g Pigment Blue 15.

### Formulierung 40:

- 14,0: demin. Wasser
- 0,3: Imidazolidinylharnstoff
- 2,5: Poloxamer 407
- 3,5: Polyvinylpyrrolidon
- 11,0: Ethanol
- 0,7: Triethanolamin
- 0,52: Carbomer
- 57,48: demineralisiertes Wasser
- 1,0: Eisenoxidpigment
- 9,0: Polymerisat PF als wässrige Dispersion

Die Komponenten werden als Gel formuliert, wobei man das Farbpigment und die wässrige Dispersion von PF zuletzt einrührt.

### Sunblock Stift

### Formulierung 41:

- 4,0: Carnauba Wax
- 4,0: Candelilla Wax
- 4,0: Bienenwachs
- 9,0: Mikrokristallines Wachs
- 1,0: Cetylpalmitat
- 10,0: Lanolin Wax
- 5,0: Ethoxyliertes Lanolin-Öl, 75 Ethylenoxideinheiten
- 5,0: Benzophenone-3
- 38,1: Capryl/Caprin-Triglycerid
- 0,2: Parfümöl
- 2,0: Titandioxid
- 0,5: Tocopherol
- 2,0: Tocopherylacetat
- 0,2: Bisabolol
- 15,0: Polymerisat PF als Pulver

Die Bestandteile der Fettmasse werden aufgeschmolzen. Dann rührt man Titandioxid ein. Bei 65 °C werden die Wirkstoffe und das pulverförmige Polymerisat PF in die Grundmasse eingearbeitet. Die homogene Schmelze wird in auf 60 °C vorgewärmte Gießformen gegossen.

### Farbige Seife:

### Formulierung 42:

- 92,9: Seifenspäne
- 2,0: Polyquaternium-16
- 0,1: Bisabolol
- 0,4: Tetrasodium EDTA
- 2,0: Parfümöl
- 1,0: PEG-6
- 1,6: Water

In 100 g der Seifengrundmasse aus genannten Bestandteilen 0,5 g wässrige Dispersion von PF einarbeiten.

### Formulierung 43:

- 4,2: Natriumhydroxid
- 5,6: Wasser
- 22,6: Propylene Glycol
- 5,2: Cocoamide DEA
- 10,4: Cocaminoxid
- 4,2: Natriumlaurylsulfat
- 7,3: Myristinsäure
- 16,6: Stearinsäure
- 5,2: Tocopherylacetat
- 18,7: Glycerin

Die Zutaten werden vermischt und bei 85 °C zu einer klaren Schmelze aufgeschmolzen. 100 Teile der Seifengrundmasse werden mit 3 Teilen wässriger Dispersion des Polymeren PF vermischt und die so erhaltene Masse noch heiß in Formen gegossen.

### Farbgebender Haarschaum (Formulierungen 44 und 45)

### Formulierung 44:

- 2,0: Cocotrimoniummethosulfat
- 0,2: Parfümöl
- 7,0: Polyquaternium-64
- 2,0: Polyquaternium-11
- 0,2: Ceteareth 25
- 0,5: Panthenol
- 0,05: Benzophenon-4
- 0,2: Mischung aus Amodimethicon, Tallowtrimoniumchlorid und Nonoxynol 10
- 0,2: Hydroxyethylcellulose
- 15,0: Ethanol
- 1,5: Polymerisat PF als wässrige Dispersion
- 10,0: Propan/Butan Wasser auf 100 g

Die Komponenten werden gemischt und zusammen mit dem Treibmittel abgefüllt.

### Formulierung 45:

- 2,0: Cocotrimoniummethosulfat
- 0,2: Parfümöl
- 6,7: Acrylsäure-Copolymer
- 0,6: Aminomethylpropanol
- 2,5: Polyvinylcaprolactam
- 0,2: Ceteareth 25
- 0,2: Panthenol
- 0,1: PEG-25 PABA
- 0,2: Hydroxyethylcellulose
- 15,0: Ethanol
- 1,0: Polymerisat PF als wässrige Dispersion
- 10,0: Propan/Butan Wasser auf 100 g

### Farbiges Haarshampoo

### Formulierung 46:

- 40,0: Natriumlaurylsulfat
- 10,0: Cocoamidopropylbetain
- q.s.: Parfümöl
- 3,0: Polyquaternium-44
- q.s.: Konservierungsmittel
- 0,5: Natriumchlorid
- 1,5: Polymerisat PF als wässrige Dispersion Wasser auf 100 g

### Sonnenschutzcreme

### Formulierung 47:

- 1,5: Ceteareth 6
- 1,0: Cetanol
- 3,0: Cetearyloctanoat
- 5,0: Polymerpulver PF 18
- 2,0: Butylmethoxydibenzoylmethan
- 6,0: Isopropylstearat
- 1,0: Glycerylstearat
- 2,0: Stearinsäure
- 3,0: Polyethylenglykol 300
- 0,3: Carbomer
- 0,6: Tetrahydroxypropylethylendiamin
- 0,1: Dinatrium-EDTA
- 0,1: Butylparaben
- 0,2: Methylparaben
- 74,2: Wasser

## Patentansprüche

1. Verwendung feinteiliger, farbstoffhaltiger Polymerisate PF in Form einer wässrigen Polymerdispersion oder eines daraus erhältlichen Polymerpulvers, dessen Polymermatrix wenigstens einen organischen Farbstoff F homogen verteilt enthält, als farbgebenden Bestandteil in kosmetischen Mitteln.

2. Verwendung nach Anspruch 1, wobei die Polymerteilchen des farbstoffhaltigen Polymerisats PF eine Teilchengröße ≤ 500 nm aufweisen.

3. Verwendung nach einem der vorhergehenden Ansprüche, wobei das farbstoffhaltige Polymerisat PF 0,5 bis 50 Gew.-%, bezogen auf das Gewicht der Polymermatrix, des organischen Farbstoffs F enthält.

4. Verwendung nach einem der vorhergehenden Ansprüche, wobei die Polymermatrix des farbstoffhaltigen Polymerisats PF aufgebaut ist aus ethylenisch ungesättigten Monomeren M.

5. Verwendung nach Anspruch 4, wobei die Monomere M umfassen:
50 bis 99,9 Gew.-% wenigstens eines monoethylenisch ungesättigten Monomeren A mit einer Wasserlöslichkeit im Bereich von 0,01 bis 60 g/l bei 25 °C,
0,1 bis 30 Gew.-% wenigstens eines vernetzenden Monomeren B,
0 bis 20 Gew.-% eines oder mehrerer monoethylenisch ungesättigter Monomere C mit einer Wasserlöslichkeit < 0,01 g/l bei 25 °C, und
0 bis 30 Gew.-% eines oder mehrerer monoethylenisch ungesättigter Monomere D mit einer Wasserlöslichkeit > 60 g/l bei 25 °C,
wobei die Mengenanteile der Monomere A, B, C und D sich zu 100 Gew.-% addieren.

6. Verwendung nach einem der Ansprüche 4 oder 5, wobei die wässrige Dispersion des farbstoffhaltigen Polymeren PF erhältlich ist durch radikalische Polymerisation einer Öl-in-Wasser-Emulsion der Monomere M, worin die Monomertröpfchen der Emulsion den organischen Farbstoff F molekulardispers verteilt enthalten.

7. Kosmetisches Mittel, enthaltend 0,1 bis 50 Gew.-%, bezogen auf das Gesamtgewicht des kosmetischen Mittels, wenigstens eines farbstoffhaltigen Polymerisats PF und für kosmetische Mittel übliche Zusätze.

8. Kosmetisches Mittel nach Anspruch 7, enthaltend zusätzlich 0,1 bis 30 Gew.-%, bezogen auf das Gesamtgewicht des kosmetischen Mittels, wenigstens eines weiteren farbgebenden Bestandteils.

9. Kosmetisches Mittel nach Anspruch 7 oder 8 in Form eines Mittels zur Behandlung der Gesichtshaut, in Form eines Mittels zur Behandlung von Augenbrauen und -wimpern, in Form eines Nagellacks, in Form eines Haarbehandlungsmittels, in Form einer Seife oder in Form einer Sonnenschutzformulierung.
